# EUROPÄISCHE PATENTSCHRIFT

(11) **EP 0 775 104 B1**
(45) Veröffentlichungstag und Bekanntmachung des Hinweises auf die Patenterteilung: **06.05.1999**
(21) Anmeldenummer: 95929815.9
(22) Anmeldetag: 08.08.1995
(51) Int. Cl.: C07C 229/26, C07C 229/36, C07C 237/08, C07C 229/22, C07C 237/06, C07C 229/16, A61K 49/00, A61K 49/04, A61K 51/04

(54) **DIMERE DTPA-DERIVATE UND DEREN METALLKOMPLEXE, DIESE KOMPLEXE ENTHALTENDE PHARMAZEUTISCHE MITTEL, DEREN VERWENDUNG IN DER DIAGNOSTIK UND THERAPIE SOWIE VERFAHREN ZUR HERSTELLUNG DER KOMPLEXE UND MITTEL**
DIMER DTPA DERIVATIVES AND THEIR METAL COMPLEXES, PHARMACEUTICALS CONTAINING THE SAME, THEIR USE IN DIAGNOSIS AND THERAPY AND PROCESS FOR PREPARING SAID COMPLEXES AND PHARMACEUTICALS
DERIVES DIMERES DE DTPA ET LEURS COMPLEXES METALLIQUES, AGENTS PHARMACEUTIQUES CONTENANT CES COMPLEXES, LEUR UTILISATION EN DIAGNOSTIC ET EN THERAPIE ET PROCEDE DE PREPARATION DE CES COMPLEXES ET AGENTS PHARMACEUTIQUES

(30) Priorität: 08.08.1994 DE 4428874
(43) Veröffentlichungstag der Anmeldung: 28.05.1997
(73) Patentinhaber: SCHERING AKTIENGESELLSCHAFT, 13342 Berlin (DE)
(72) Erfinder: KRAUSE, Werner, D-13505 Berlin (DE); MAIER, Franz-Karl, D-13467 Berlin (DE); BAUER, Michael, D-13505 Berlin (DE); PRESS, Wolf-Rüdiger, D-12103 Berlin (DE); SCHUHMANN-GIAMPIERI, Gabriele, D-12203 Berlin (DE); PLATZEK, Johannes, D-12621 Berlin (DE); SCHMITT-WILLICH, Heribert, D-12161 Berlin (DE)
(86) Internationale Anmeldenummer: EP9503142
(87) Internationale Veröffentlichungsnummer: WO9605167

(56) Entgegenhaltungen:
- WO-A-88/07521
- WO-A-91/05762
- JOURNAL OF GENERAL CHEMISTRY USSR, Bd.60, Nr.2, 1990, NEW YORK US Seiten 309 - 313 V. G. YASHUNSKII ET AL 'Synthesis, acid-base characteristics, and complex-forming properties of [[(2-hydroxy trimethylene)dinitrilo]tetrakis(ethylenedi nitrilo)]-octaacetic acid'

## Beschreibung

Die Erfindung betrifft die in den Ansprüchen gekennzeichneten Gegenstände, das heißt neue dimere DTPA-Derivate und deren Metallkomplexe, diese Komplexe enthaltende pharmazeutische Mittel, deren Verwendung in der Diagnostik und Therapie sowie Verfahren zur Herstellung der Komplexe und Mittel.

Kontrastmittel sind unentbehrliche Hilfsmittel in der modernen Diagnostik; so wären viele Erkrankungen ohne den Einsatz von Kontrastmitteln nicht diagnostizierbar. Kontrastmittel finden in allen Bereichen der Diagnostik, wie z.B. der Röntgen-, der Radio- oder der Ultraschalldiagnostik oder der magnetischen Resonanztomographie, Verwendung.

Die Wahl der jeweils bevorzugten Methode richtet sich u.a. nach der diagnostischen Fragestellung, wird aber auch durch die dem Mediziner jeweils zur Verfügung stehenden apparativen Möglichkeiten bestimmt. So hat insbesondere die Kernspintomographie aufgrund des erheblichen technischen und damit verbunden hohen Kostenaufwands noch nicht die weite Verbreitung der anderen Methoden, wie z.B. röntgendiagnostischer Methoden, gefunden.

Auch die Wahl des geeigneten Kontrastmittels variiert in Abhängigkeit der jeweiligen Fragestellung. So wird die Eignung des Kontrastmittels für eine bestimmte Aufgabe nicht zuletzt durch sein (Anreicherungs-) Verteilungsverhalten im Organismus bestimmt.

Obgleich sowohl auf der gerätetechnischen als auch auf der Kontrastmittelseite große Fortschritte erzielt worden sind, stehen noch nicht für alle Fragestellungen befriedigende Lösungen zur Verfügung.

So gibt es für die verschiedenen bildgebenden Verfahren nicht für alle Indikationen die geeigneten Kontrastmittel. Dies gilt insbesondere für die Computertomographie.

Paramagnetische Metallkomplexe mit Aminopolycarbonsäuren sind im US-Patent 4,647,447 (Gries et al.) als Kontrastmittel für die NMR-Diagnostik vorgeschlagen worden, von denen sich der Gadolinium-Komplex der Diethylentriaminpentaessigsäure (Gd-DTPA) als Diagnostikum Magnevist® besonders bewährt hat [H.P. Niendorf et al., Advances in MRI Contrast (1993); 2: 12-19]. Es wurde auch bereits vorgeschlagen, diesen einkernigen Metallkomplex als Röntgenkontrastmittel zu verwenden [C. Zwicker et al., Fortschr. Röntgenstr. 158, 3 (1993), 255-259], insbesondere für Patienten, die eine Überempfindlichkeit gegenüber den konventionell verwendeten iodhaltigen Kontrastmitteln zeigen [Y. Kinno et al., A.J.R. (1993); 160: 1293-1294]. Im Hinblick auf den höheren Massenschwächungskoeffizienten, z.B. der Lanthaniden gegenüber Iod, aber der geringeren Absorption eines Lanthanidatoms im Vergleich zu einem Triiodaromaten, erscheint die Synthese von Liganden, die in der Lage sind, mehr als ein Metallatom pro Molekül stabil zu binden, von großem Interesse. Die bisherigen Bemühungen in diese Blickrichtung haben noch nicht zu befriedigenden Resultaten geführt. So weisen die in der WO 91/05762 [D. Love et al.] beschriebenen Verbindungen eine ungenügende Hitze- und Langzeit. stabilität der Komplexsalzlösungen auf. Auch lassen diese Komplexe bezüglich ihrer Wasserlöslichkeit Wünsche offen. Dies gilt auch für die in der WO 88/07521 [J. Deutsch et. al.] vorgeschlagenen Verbindungen, deren Verträglichkeit noch nicht befriedigend ist.

In Journal of General Chemistry USSR, Vol. 60, Nr. 2, 1990, 309-313 wird die Bestimmung von Stabilitätskonstanten der Komplexe von {[(2-Hydroxytrimethylen)dinitrilo]-tetrakis(ethylendinitrilo)}-octaessigsäure mit veschiedenen Metallen beschrieben. Es findet sich in diesem Dokument kein Hinweis darauf, daß mit diesen - nicht von der Definition der allgemeinen Formel I umfaßten - Verbindungen die dieser Erfindung zugrundeliegenden Aufgaben gelöst werden können.

Es wurde nun gefunden, daß die erfindungsgemäßen, in ihrer Struktur neuartigen Liganden Metallkomplexe bilden, die nicht nur eine hohe Wasserlöslichkeit, sondern darüberhinaus auch eine überraschend hohe Verträglichkeit zeigen und damit die Voraussetzungen insbesondere für ein für Bolusinjektionen geeignetes Röntgenkontrastmittel erfüllen. Sie sind aber auch für die NMR-Diagnostik und für die Radiodiagnostik und Radiotherapie als Kontrastmittel hervorragend geeignet.

Die neuen erfindungsgemäßen Verbindungen werden durch die allgemeine Formel I - bestehend aus den Inkrementen (A) und (B) - beschrieben: 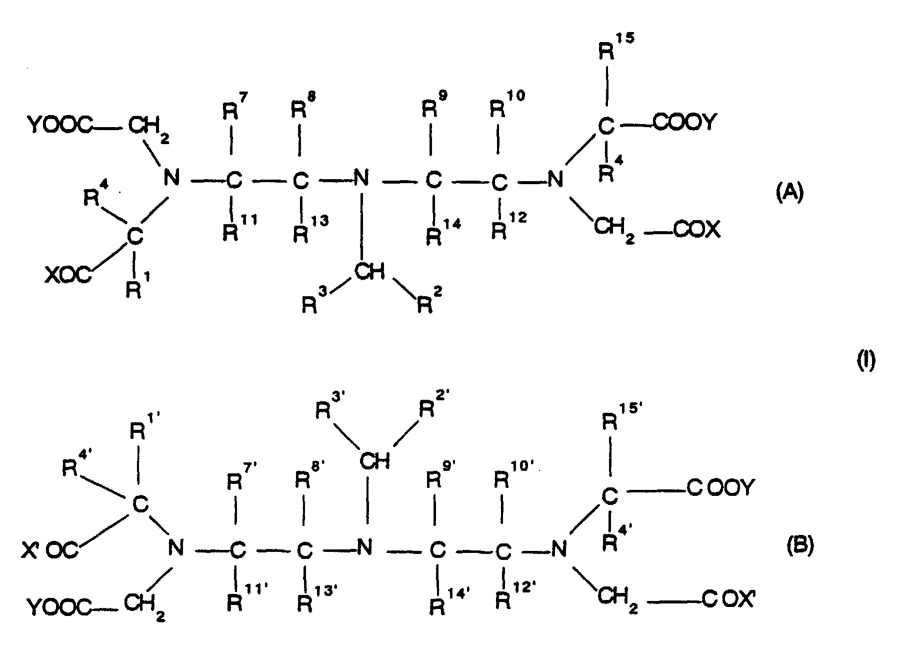
wobei die Inkremente A und B über eines der Substituentenpaare R¹ und R^{1'}, R² und R^{2'}, R¹⁵ und R^{15'}, R¹ und R^{2'}, R^{1'} und R², R^{1'} und R¹⁵, R¹ und R¹⁵, R² und R^{15'}, R^{2'} und R¹⁵ miteinander verknüpft sind,
worin das jeweils verknüpfende Substituentenpaar für einen Heterocyclus, einen Phenylenrest, eine C₀ - C₃₀-Alkylen- oder eine C₇- C₃₀ Aralkylenkette stehen, die gegebenenfalls durch 1 - 4 Hydroxy-, C₁ - C₆-Alkoxy-, Carboxy- oder Mercaptogruppen substituiert und/oder durch 1 bis 6 Sauerstoff-, Stickstoff-, Schwefelatome, Sulfinyl- und/oder Sulfonylgruppen unterbrochen ist,
und die für die Verknüpfung nicht benötigten Substituenten R¹, R^{1'}, R¹⁵ und R^{15'} unabhängig voneinander für ein Wasserstoffatom oder eine gegebenenfalls durch 1 - 4 Hydroxy- oder Mercaptogruppen substituierte C₁ - C₆-Alkylgruppe stehen, und die für die Verknüpfung nicht benötigten Substituenten R² und R^{2'} unabhängig voneinander für ein Wasserstoffatom, eine C₁ - C₆-Alkylgruppe, eine Gruppe -(CH₂)ₙOH oder -(CH₂)ₙSH mit n = 1 oder 2 stehen,
und die Substituenten R³ und R^{3'} unabhängig voneinander für eine Gruppe COOY-, oder für eine CONR⁵R⁶-Gruppe stehen, worin R⁵ und R⁶ unabhängig voneinander eine C₁ - C₆-Alkylgruppe, die gegebenenfalls durch 1 - 4 Hydroxy- oder C₁ - C₆-Alkoxygruppen substituiert und/oder durch 1 - 6 Sauerstoff-, Stickstoff- und/oder Schwefelatome unterbrochen ist, bedeuten oder gemeinsam unter Einbeziehung des Stickstoffatoms einen 5- oder 6-Ring bilden, der gegebenenfalls ein Sauerstoffatom, ein weiteres acyliertes Stickstoffatom oder eine Sulfonylgruppe enthält und/oder mit 1 - 3 Hydroxygruppen substituiert ist
und die Substituenten R⁴ und R^{4'} für ein Wasserstoffatom und/oder eine C₁ - C₆-Alkylgruppe stehen,
und die Substituenten R⁷ - R¹⁴ und R^{7'} - R^{14'} für ein Wasserstoffatom, eine Phenylgruppe, eine C₀ - C₃₀-Alkylen- oder eine C₇- C₃₀ Aralkylenkette stehen, die gegebenenfalls durch 1 - 4 Hydroxy-, C₁ - C₆-Alkoxy- oder Mercaptogruppen substituiert und/oder durch 1 - 6 Sauerstoff-, Stickstoff- und/oder Schwefelatome unterbrochen ist,
oder die Substituenten R⁷ und R⁸, R^{7'} und R^{8'} oder R⁹ und R¹⁰ und R^{9'} und R^{10'} gemeinsam eine Tri- oder Tetramethylengruppe bilden,
und Y jeweils für ein Wasserstoffatom und/oder ein Metallionenäquivalent eines Elements der Ordnungszahlen 21 - 32, 37 - 39, 42 - 51 und 57 - 83 steht,
und X und X' für eine Gruppe -OY, oder -CONR⁵R⁶ stehen, mit Y, R⁵ und R⁶ in der genannten Bedeutung,
sowie deren Salze mit anorganischen und/oder organischen Basen oder Aminosäuren.

Verbindungen der allgemeinen Formel I - bestehend aus den Inkrementen (A) und (B) - mit Y in der Bedeutung von Wasserstoff werden als Komplexbildner und mit mindestens zwei der Substituenten Y in der Bedeutung eines Metallionenäquivalents als Metallkomplexe bezeichnet.

Unter einer C₀-Alkylenkette wird eine direkte Bindung verstanden.

Die Elemente der genannten Ordnungszahlen umfassen (je nach angestrebten Verwendungszweck) auch deren radioaktive Isotope.

Ist das erfindungsgemäße Mittel zur Anwendung in der NMR-Diagnostik bestimmt, so muß das Metallion des Komplexsalzes paramagnetisch sein. Dies sind insbesondere die zwei- und dreiwertigen Ionen der Elemente der Ordnungszahlen 21 - 29, 42, 44 und 58 - 70. Geeignete Ionen sind beispielsweise das Chrom(III)-, Eisen(II)-, Cobalt(II)-, Nickel(II)-, Kupfer(II)-, Praseodym(III)-, Neodym(III)-, Samarium(III)- und Ytterbium(III)-ion. Wegen ihres starken magnetischen Moments sind besonders bevorzugt Gadolinium(III)-, Terbium(III)-, Dysprosium(III)-, Holmium(III)-, Erbium(III)-, Eisen(III)- und Mangan(II)-ionen.

Für die Verwendung der erfindungsgemäßen Mittel in der Nuklearmedizin muß das Metallion radioaktiv sein. Geeignet sind zum Beispiel Radioisotope der Elemente Kupfer, Kobalt, Gallium, Germanium, Yttrium, Strontium, Technetium, Indium, Ytterbium, Gadolinium, Samarium, Iridium, Rhenium und Bismut; bevorzugt sind Technetium, Gallium, Indium und Rhenium.

Ist das erfindungsgemäße Mittel zur Anwendung in der Röntgen-Diagnostik bestimmt, so leitet sich das Metallion vorzugsweise von einem Element höherer Ordnungszahl ab, um eine ausreichende Absorption der Röntgenstrahlen zu erzielen. Es wurde gefunden, daß zu diesem Zweck diagnostische Mittel, die ein physiologisch verträgliches Komplexsalz mit Metallionen von Elementen der Ordnungszahlen 25 und 26 sowie 57 - 83 enthalten, geeignet sind.

Bevorzugt sind Mangan(II)-, Eisen(II)-, Eisen(III)-, Praseodym(III)-, Neodym(III)-, Samarium(III)-, Gadolinium(III)-, Ytterbium(III)- oder Bismut(III)-ionen, insbesondere Dysprosium(III)-ionen.

Die Verknüpfung der Inkremente (A) und (B) erfolgt jeweils über eines der Substituentenpaare R¹ und R^{1'}, R² und R^{2'}, R¹⁵ und R^{15'}, R¹ und R^{2'}, R^{1'} und R², R^{1'} und R¹⁵, R¹ und R^{15'}, R² und R^{15'}, R^{2'} und R¹⁵.
Bevorzugt erfolgt die Verknüpfung über die Substituentenpaare R¹ und R^{1'}, R² und R^{2'} und R¹ und R^{2'}.
Diese Brückenglieder bestehen aus C₀ - C₃₀-Alkylen- oder C₇- C₃₀-Aralkylenketten, die durch 1 - 4 Hydroxy-, C₁ - C₆-Alkoxy-, Carboxy- oder Mercaptogruppen substituiert und/oder durch 1 - 6 Sauerstoff-, Stickstoff- oder Schwefelatom oder durch Sulfinyl- oder Sulfonylgruppen unterbrochen sein können.

Für das Substituentenpaar R² und R^{2'} sind die direkte Bindung, die Methylen-, Dimethylen- und Trimethylen-, die Benzylmethylen- und Benzylethergruppen bevorzugt.

Die für die Verknüpfung nicht benötigten Substituenten R¹, R^{1'}, R¹⁵, R^{15'} können unabhängig voneinander Wasserstoffatome und/oder gegebenenfalls durch 1 - 4 Hydroxy- oder Mercaptogruppen substituierte C₁ - C₆-Alkylgruppen sein, die Substituenten R² und R^{2'} können zusätzlich für eine Gruppe -(CH₂)ₙOH oder -(CH₂)ₙSH mit n = 1 oder 2 stehen.

Die Substituenten R³ und R^{3'} können unabhängig voneinander für Wasserstoffatome, für eine COOY- Gruppe, für eine C₀ - C₃₀-Alkylen- oder eine C₇- C₃₀-Aralkylenkette, die gegebenenfalls durch 1 - 4 Hydroxy- oder C₁ - C₆-Alkoxygruppen substituiert und/oder durch 1 - 6 Sauerstoff-, Stickstoff- und/oder Schwefelatome unterbrochen ist oder für eine CONR⁵R⁶-Gruppe stehen, worin R⁵ und R⁶ gleich oder verschieden sind und eine C₁ - C₆-Alkylgruppe, die gegebenenfalls durch 1 - 4 Hydroxy- oder C₁ - C₆-Alkoxygruppen substituiert und/oder durch 1 - 6 Sauerstoff-, Stickstoff- und/oder Schwefelatom unterbrochen ist, bedeuten, oder gemeinsam unter Einbeziehung des Stickstoffatoms einen 5- oder 6-Ring bilden, der gegebenenfalls ein Sauerstoffatom, ein weiteres acyliertes Stickstoffatom oder eine Sulfonylgruppe enthält und/oder mit 1 - 3 Hydroxygruppen substituiert ist. Beispielhaft genannt seien 3,4-Dihydroxypyrrolidin oder (S,S-Dioxo)-thiomorpholin.

Bevorzugte Substituenten R³/R^{3'} sind die Carboxyl- und Carboxamidgruppen.

Die Substituenten R⁴ und R^{4'} stehen für Wasserstoffatome und/oder C₁ - C₆-Alkylgruppen, wobei Wasserstoffatome und Methylgruppen bevorzugt sind.

Die Substituenten R⁷, R⁸, R⁹, R¹⁰, R¹¹, R¹², R¹³, R¹⁴, R^{4'}, R^{8'}, R^{9'}, R^{10'}, R^{11'}, R^{12'}, R^{13'} und R^{14'} stehen für ein Wasserstoffatom, eine Phenylgruppe oder eine C₀ - C₃₀-Alkylen- oder Aralkylenkette, die gegebenenfalls durch 1 - 4 Hydroxy-, C₁ - C₆-Alkoxy- oder Mercaptogruppen substituiert und/oder durch 1 - 6 Sauerstoff-, Stickstoff- oder Schwefelatome unterbrochen ist.
Die Substituenten R⁷ und R⁸, R^{7'} und R^{8'}, R⁹ und R¹⁰ und R^{9'} und R¹⁰ können darüber hinaus jeweils gemeinsam eine Tri- oder Tetramethylengruppe bilden.

Bevorzugte Substituenten R⁷ - R¹⁴ und R^{7'} - R^{14'} sind Wasserstoffatome, Methyl-, Ethyl-und/oder Hydroxymethylgruppen.

Die Substituenten X und X' können sowohl für einen Rest -OY als auch für einen Rest -CONR⁵R⁶- (mit R⁵/R⁶ in der genannten Bedeutung) stehen.

Y steht für ein Wasserstoffatom oder ein Metallionenäquivalent eines Elements der Ordnungszahlen 21 - 32, 37 - 39, 42 - 51 und 57 - 83.

Die Herstellung der erfindungsgemäßen Metallkomplexe erfolgt in der Weise, wie sie in der Patentanmeldung DE-OS 34 01 052 offenbart worden ist, indem man das Metalloxid oder ein Metallsalz (beispielsweise das Nitrat, Acetat, Carbonat, Chlorid oder Sulfat) des Elements der Ordnungszahlen 21 - 32, 37 - 39, 42- 51, 57 - 83 in Wasser und/oder einem polaren organischen Lösungsmittel, wie z.B. DMF und/oder Methanol, Ethanol oder Isopropanol löst oder suspendiert und mit der Lösung oder Suspension der äquivalenten Menge der komplexbildenden Säure der allgemeinen Formel I mit Y in der Bedeutung eines Wasserstoffatoms umsetzt und anschließend gewünschtenfalls vorhandene azide Wasserstoffatome durch Kationen anorganischer und/oder organischer Basen oder Aminosäuren substituiert.

Die Neutralisation erfolgt dabei mit Hilfe anorganischer Basen (zum Beispiel Hydroxiden, Carbonaten oder Bicarbonaten) von zum Beispiel Natrium, Kalium, Calcium oder Zink und/oder organischer Basen wie unter anderem primärer, sekundärer und tertiärer Amine, wie zum Beispiel Ethanolamin, Morpholin, Glucamin, N-Methyl- und N,N-Dimethylglucamin, sowie basischer Aminosäuren, wie zum Beispiel Lysin, Arginin und Omithin.

Zur Herstellung der neutralen Komplexverbindungen kann man beispielweise den sauren Komplexsalzen in wäßriger Lösung oder Suspension so viel der gewünschten Basen zusetzen, daß der Neutralpunkt erreicht wird. Die erhaltene Lösung kann anschließend im Vakuum zur Trockne eingeengt werden. Häufig ist es von Vorteil, die gebildeten Neutralsalze durch Zugabe von mit Wasser mischbaren Lösungsmitteln, wie zum Beispiel niederen Alkoholen (Methanol, Ethanol, Isopropanol und anderen), niederen Ketonen (Aceton und andere), polaren Ethern (Tetrahydrofuran, Dioxan, 1,2-Dimethoxyethan und andere) auszufällen und so leicht zu isolierende und gut zu reinigende Kristallisate zu erhalten. Als besonders vorteilhaft hat es sich erwiesen, die gewünschte Base bereits während der Komplexbildung der Reaktionsmischung zuzusetzen und dadurch einen Verfahrensschritt einzusparen.

Enthalten die sauren Komplexverbindungen mehrere freie azide Gruppen, so ist es oft zweckmäßig, neutrale Mischsalze herzustellen, die sowohl anorganische als auch organische Kationen als Gegenionen enthalten.

Dies kann beispielsweise geschehen, indem man die komplexbildende Säure in wäßriger Suspension oder Lösung mit dem Oxid oder Salz des das Zentralion liefernden Elements und der Hälfte der zur Neutralisation benötigten Menge einer organischen Base umsetzt, das gebildete Komplexsalz isoliert, es gewünschtenfalls reinigt und dann zur vollständigen Neutralisation mit der benötigten Menge anorganischer Base versetzt. Die Reihenfolge der Basenzugabe kann auch umgekehrt werden.

Im Falle der Verwendung von Radioisotope enthaltenden Komplexverbindungen kann deren Herstellung nach den in "Radiotracers for Medical Applications", Volume 1, CRC-Press, Boca Raton, Florida, beschriebenen Methoden vorgenommen werden.

Die Herstellung der erfindungsgemäßen Verbindungen mit Y in der Bedeutung eines Wasserstoffatoms kann auf vielfältige Art und Weise erfolgen. Die verschiedenen Verfahren sind dem Fachmann prinzipiell bekannt.

So kann man Verbindungen der allgemeinen Formel I zum Beispiel dadurch erhalten, daß man die beiden primären Aminogruppen einer Diaminodicarbonsäure der allgemeinen Formel II 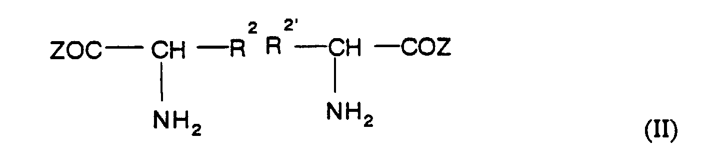 worin
R²R^{2'} eine direkte Bindung oder eine C₁ - C₃₀-Alkylen- oder eine C₇- C₃₀-Aralkylenkette bedeutet, die gegebenenfalls durch Hydroxy-, C₁ - C₆-Alkoxy- oder Mercaptogruppen substituiert und/oder durch Heteroatome unterbrochen ist und
Z für eine Gruppe -NR⁵R⁶, worin R⁵ und R⁶ die genannte Bedeutung haben, oder für eine funktionelle Gruppe -OG steht, worin G ein Wasserstoffatom oder eine Schutzgruppe, wie beispielsweise eine tert.-Butyl- oder Benzylgruppe, bedeutet,
wobei gegebenenfalls vorhandene Hydroxygruppen beispielsweise als Acetale geschützt sind, mit Verbindungen der allgemeinen Formel III 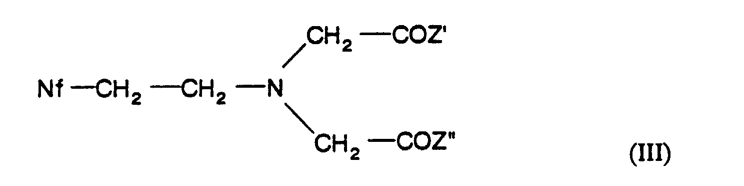 umsetzt, worin
Nf ein Nucleofug, wie beispielsweise ein Chlorid, Bromid oder Iodid bedeuten und
Z' und Z" unabhängig voneinander die für Z angegebene Bedeutung haben
und daß man nach Abspaltung der Schutzgruppen eine Polycarbonsäure der allgemeinen Formel IV 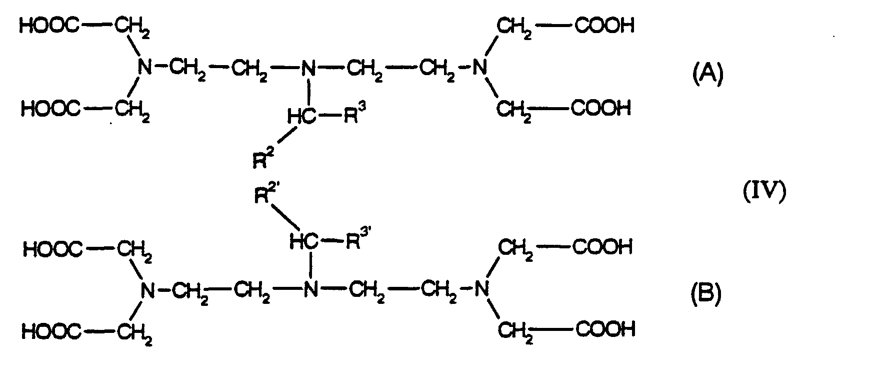 erhält, mit R²R^{2'} in der Bedeutung einer C₀ - C₃₀-Alkylen- oder einer C₇- C₃₀-Aralkylenkette, die gegebenenfalls durch 1 - 4 Hydroxy-, C₁ - C₆-Alkoxy-, Carboxy- oder Mercaptogruppen substituiert und/oder durch Sauerstoff-, Stickstoff- und/oder Schwefelatome oder durch eine Sulfinyl- oder Sulfonylgruppe unterbrochen ist und mit R³ und R^{3'} in der genannten Bedeutung und die Verbindungen der Formel IV anschließend gewünschtenfalls in ein Salz einer anorganischen und/oder organischen Base oder Aminosäure umwandelt. Gewünschtenfalls kann ein Teil der Carboxygruppen nach deren Aktivierung und Umsetzung mit einem Amin der allgemeinen Formel V

HNR⁵R⁶ (V)

mit R⁵ und R⁶ in der genannten Bedeutung, in Amidgruppen überführt werden.

Wird die Synthese ausgehend von einer schwefelhaltigen Diaminodicarbonsäure der allgemeinen Formel II in der Bedeutung von beispielsweise Cystathionin oder Lanthionin durchgeführt, so können die Thioethergruppen gewünschtenfalls durch geeignete Oxidationsmittel, wie Wasserstoffperoxid oder Persäuren, vorzugsweise vor Abspaltung gegebenenfalls vorhandener Schutzgruppen in die entsprechenden Sulfinyl- oder Sulfonylverbindungen in an sich bekannter Weise überführt werden.

Zu Verbindungen der allgemeinen Formel IV gelangt man auch, indem man eine Dicarbonsäure der allgemeinen Formel VI 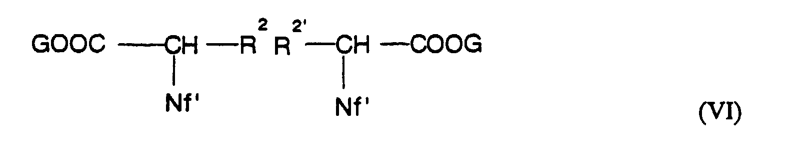 worin
R²R^{2'} und G die oben genannte Bedeutung haben und Nf' für ein Nucleofug, wie z.B. ein Chlorid, Bromid, Iodid, O-Mesylat, O-Tosylat oder O-Triflat, steht,
mit dem Bis-(2-phthalimidoethyl)-amin zur Tetraphthalimidoverbindung der allgemeinen Formel VII 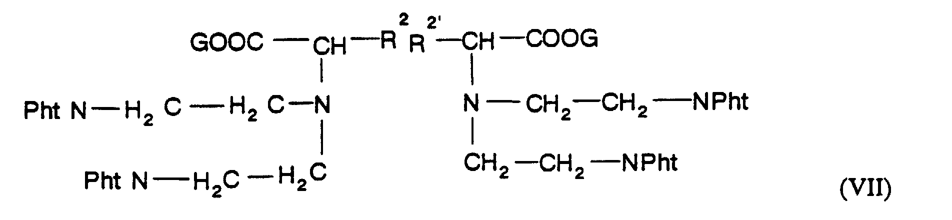 worin R²R^{2'} die genannte Bedeutung hat und NPht für eine Phthalimidogruppe steht, in an sich bekannter Weise umsetzt.

Nach Abspaltung dieser Schutzgruppen erhält man ein Dipiperazinoderivat der allgemeinen Formel VIII 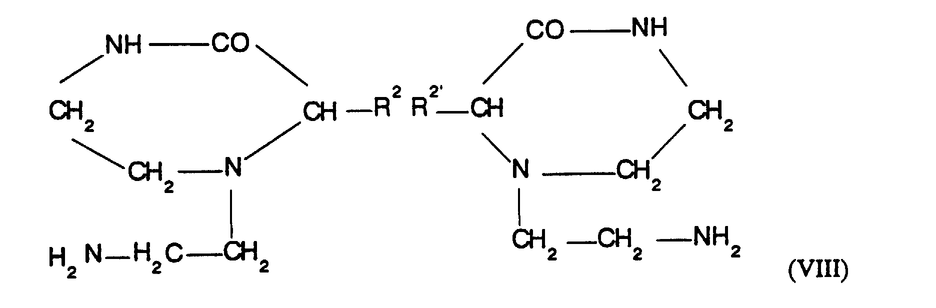 mit R²R^{2'} in der oben genannten Bedeutung, aus dem mit einem Reaktanten der allgemeinen Formel IX

Nf-CH₂-COOG' (IX)

mit Nf in der Bedeutung von Chlorid, Bromid oder Iodid und G' in der Bedeutung von G mit der Ausnahme von Wasserstoff, nach Abspaltung der Schutzgruppen eine Verbindung der allgemeinen Formel X 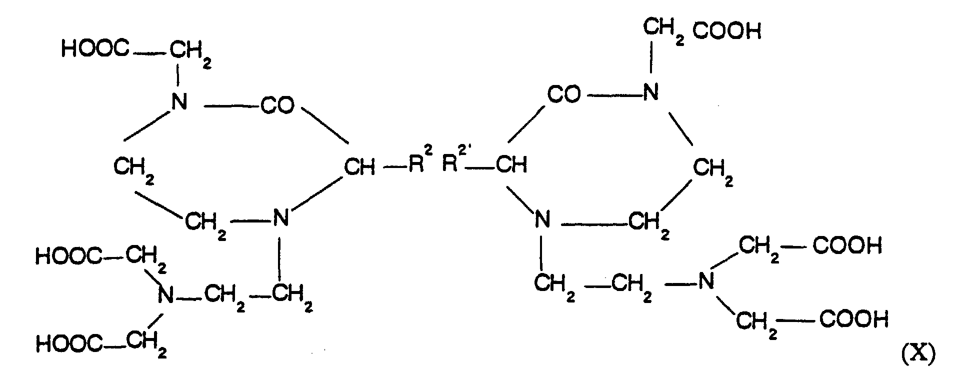 mit R²R^{2'} in der genannten Bedeutung, erhalten wird.

Die erhaltene Hexacarbonsäure wird anschließend nacheinander einer Lactamspaltung und einer N-Alkylierung mit einem Reaktanten der allgemeinen Formel XI,

Nf-CH₂-COOH (XI)

worin Nf die genannte Bedeutung hat, unterworfen, wobei eine Verbindung der allgemeinen Formel IV, mit R²R^{2'} in der genannten Bedeutung und R³ und R^{3'} in der Bedeutung einer Carboxylgruppe, erhalten wird.

Eine andere Art der Syntheseführung besteht darin, daß man ausgehend von einer Hydroxy- oder Mercaptogruppe(n) tragenden, gegebenenfalls als Ester vorliegenden Aminosäure, wie beispielsweise L-Tyrosinbenzylester, durch Alkylierung mit einer Verbindung der allgemeinen Formel III eine Verbindung der allgemeinen Formel XII 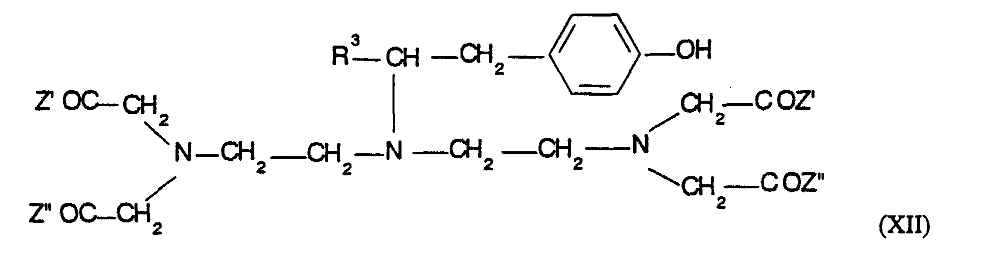 mit R³, Z' und Z" in der oben genannten Bedeutung, herstellt und anschließend durch O-Alkylierung mit einem bifunktionellen Alkylierungsmittel, wie beispielsweise 1,8-Diiodoctan, jeweils zwei Moleküle der allgemeinen Formel XII etherartig verknüpft und nachfolgend gegebenenfalls vorhandene Schutzgruppen abspaltet.

Bei einer anderen Art der Syntheseführung geht man von einem Diamin der allgemeinen Formel XIII aus 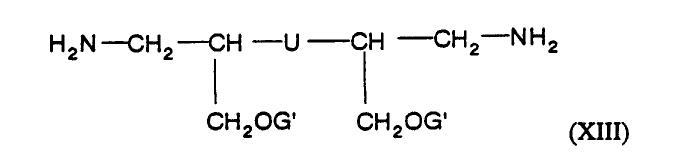 mit G' in der oben genannten Bedeutung einer Schutzgruppe für Hydroxyfunktionen und U in der Bedeutung einer direkten Bindung oder einer C₁ - C₃₀-Alkylen- oder einer C₇- C₃₀-Aralkylenkette, die gegebenenfalls durch 1 - 4 Hydroxy-, C₁ - C₆-Alkoxy- oder Mercaptogruppen substituiert und/oder durch 1 - 6 Sauerstoff-, Stickstoff- oder Schwefelatome unterbrochen ist. Dieses wird mit einem Elektrophil der allgemeinen Formel III alkyliert. Abschließend spaltet man gegebenenfalls vorhandene Schutzgruppen in üblicher Weise ab.

Bei einer anderen Variante der Syntheseführung erhält man ausgehend von einer Verbindung der Formel II, mit R²R^{2'} und Z in der oben genannten Bedeutung, durch Alkylierung mit einer Verbindung der allgemeinen Formel XIV 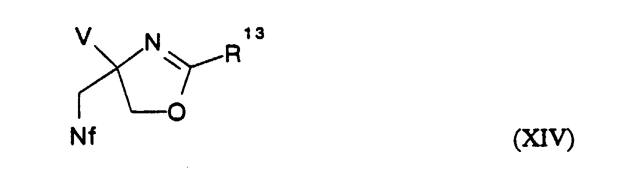 worin R¹³ eine C₁ - C₆-Alkylpqppe, V eine Gruppe R¹¹, R^{11'}, R¹² oder R^{12'} bedeuten und Nf die genannte Bedeutung hat, ein Tetraoxazolin-Derivat. Verbindungen der Formel XIV sind beispielsweise aus einem Serinol-Derivat der allgemeinen Formel XV erhältlich 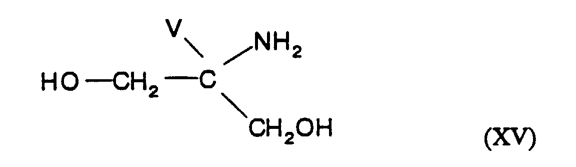 worin V die genannte Bedeutung hat.
Aus dem Tetraoxazolin-Derivat wird durch Hydrolyse ein Hexamin der allgemeinen Formel XVI 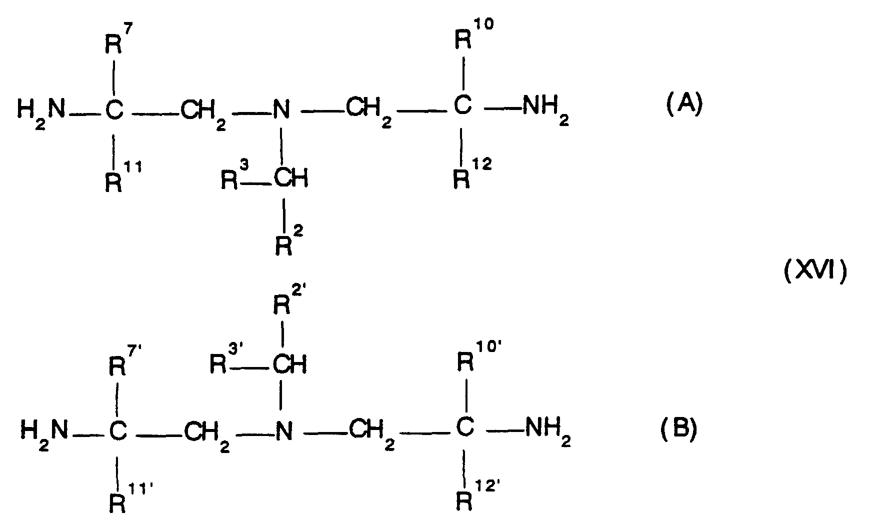 erhalten, worin R²R^{2'} eine direkte Bindung oder eine C₁ - C₃₀-Alkylenkette oder einer C₇- C₃₀-Aralkylenkene, die gegebenenfalls durch Hydroxy-, C₁ - C₆-Alkoxy- oder Mercaptogruppen substituiert und/oder durch 1 - 6 Sauerstoff-, Stickstoff- oder Schwefelatome unterbrochen ist, bedeutet und worin R³, R^{3'}, R⁷, R^{7'}, R¹⁰, R^{10'}, R¹¹, R^{11'}, R¹², R^{12'} die genannten Bedeutung haben. Das Hexamin der Formel XVI wird anschließend mit einer Halogenessigsäure, die gegebenenfalls in geschützter Form, beispielsweise als tert.-Butylesters vorliegt, alkyliert. Vorhandene Schutzgruppen werden anschließend in an sich bekannter Weise abgespalten.

Bei einer anderen Art der Syntheseführung geht man von einem Diamin der allgemeinen Formel II aus und erhält durch Umsetzung der Aminogruppen mit einem aromatischen Aldehyd, wie beispielsweise Benzaldehyd, und anschließender Reduktion, beispielsweise mit Natriumcyanoborhydrid, ein N,N'-geschütztes Diamin von der Art, wie es beispielsweise auch durch Umsetzung einer α,α'-Dihalogendicarbonsäure mit z.B. Benzylamin erhalten werden kann. Nach N,N'-Dialkylierung mit einer Verbindung der allgemeinen Formel III und hydrogenolytischer Abspaltung der aromatischen Schutzgruppen resultiert eine Verbindung der allgemeinen Formel XVII 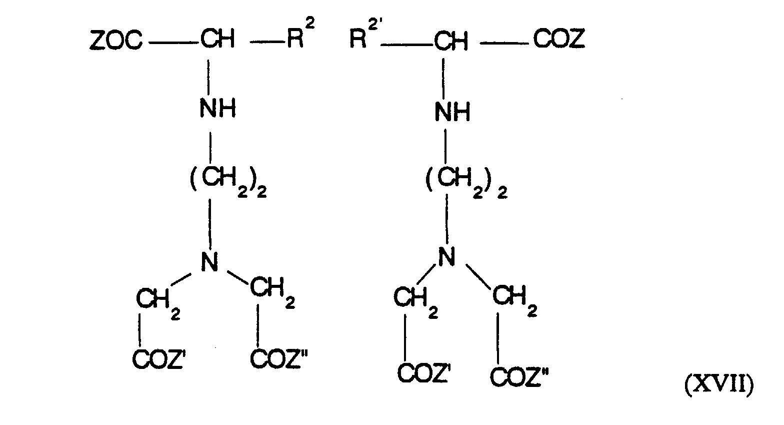 worin R²R^{2'}, Z, Z' und Z" die genannten Bedeutungen haben.
Die Verbindung der Formel XVII wird mit einem geeigneten Liganden, der aus einem C-substituierten β-Aminoalkohol erhältlich ist, der zunächst mit einer Halogenessigsäure in der üblichen Weise N-alkyliert und dessen Hydroxygruppe in an sich bekannter Weise z.B. in ein Bromid überführt wird, zu den erfindungsgemäßen Komplexbildnern umgesetzt.

Bei einer anderen Synthesevariante geht man von einer substituierten DTPA (Diethylentriaminpentaessigsäure) der allgemeinen Formel XVIII 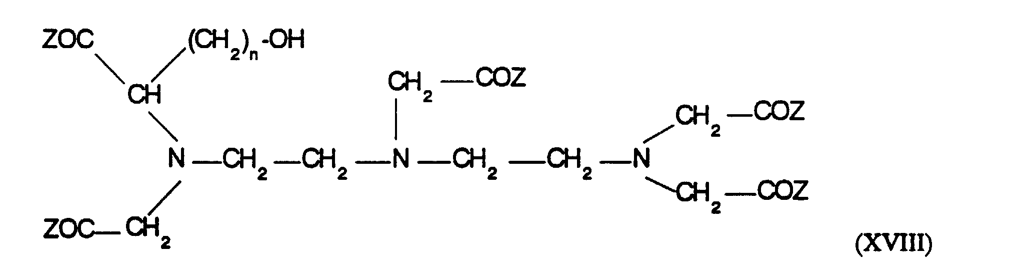 aus, worin Z und n die oben genannte Bedeutung haben.
Derartige Verbindungen sind beispielsweise in dem Fachmann bekannter Weise durch hydrogenolytische Spaltung eines im Beispiel 4b) der EP-A 0 230 893 beschriebenen Benzylethers erhältlich. Nach Umwandlung der Hydroxylgruppe in ein Nucleofug Nf (wie z.B. ein Bromid, Mesylat oder Tosylat) erhält man einen alkylierenden Reaktanten, der mit einem Phenol der allgemeinen Formel XII oder mit einem Alkohol der allgemeinen Formel XVIII umgesetzt wird. Nach Abspaltung gegebenenfalls vorhandener Schutzgruppen erhält man die erfindungsgemäßen Komplexbildner der allgemeinen Formel I mit Y in der Bedeutung von Wasserstoff.

Zu weiteren Verbindungen der allgemeinen Formel I gelangt man dadurch, daß man eine Diaminoverbindung der allgemeinen Formel II, XIII oder XVII mit einem gegebenenfalls mit R⁷, R⁸, R¹³ bzw. R⁹, R¹⁰, R¹⁴ substituierten Nitroethen nach Art einer Michael-Addition umsetzt, die Nitrogruppen beispielsweise mit Samariumdiiodid reduziert [M.A. Sturgess et al., Tetrahedron Lett. 34 (1993): 4743 - 4746] und die erzeugten Aminogruppen in üblicher Weise mit einem Reaktanten der allgemeinen Formel IX alkyliert und abschließend gegebenenfalls vorhandene Schutzgruppen abspaltet.

Zu Thiol-Gruppen enthaltenden Verbindungen der allgemeinen Formel I gelangt man nach dem oben beschriebenen Verfahren, wobei die in Vorstufen enthaltenen Thiol-Gruppen in der Regel in geschützter Form vorliegen und in einem der letzten Reaktionsschritte freigesetzt werden (vgl. T.W. Green und P.G.M. Wuts, Protective Groups in Organic Synthesis, J. Wiley & Sons Inc., New York).

Die Herstellung der erfindungsgemäßen pharmazeutischen Mittel erfolgt ebenfalls in an sich bekannter Weise, indem man die erfindungsgemäßen Komplexverbindungen - gegebenenfalls unter Zugabe der in der Galenik üblichen Zusätze - in wäßrigem Medium suspendiert oder löst und anschließend die Suspension oder Lösung gegebenenfalls sterilisiert. Geeignete Zusätze sind beispielsweise physiologisch unbedenkliche Puffer (wie zum Beispiel Tromethamin), geringe Zusätze von Komplexbildnern (wie zum Beispiel DTPA oder der jeweiligen erfindungsgemäßen Verbindung der allgemeinen Formel I mit Y in der Bedeutung von Wasserstoff) und/oder deren Calcium-, Magnesium- oder Zinksalze oder gewünschtenfalls Elektrolyte (wie zum Beispiel Natriumchlorid) sowie Antioxidantien (wie zum Beispiel Ascorbinsäure).

Sind für die enterale Verabreichung oder andere Zwecke Suspensionen oder Lösungen der erfindungsgemäßen Mittel in Wasser oder physiologischer Salzlösung erwünscht, werden sie mit einem oder mehreren in der Galenik üblichen Hilfsstoff(en) (zum Beispiel Methylcellulose, Lactose, Mannit) und/oder Tensid(en) (zum Beispiel Lecithine, Tween®, Myrj®) und/oder Aromastoff(en) zur Geschmackskorrektur (zum Beispiel ätherischen Ölen) gemischt.

Prinzipiell ist es auch möglich, die erfindungsgemäßen pharmazeutischen Mittel auch ohne Isolierung der Komplexsalze herzustellen. In jedem Fall muß besondere Sorgfalt darauf verwendet werden, die Chelatbildung so vorzunehmen, daß die erfindungsgemäßen Salze und Salzlösungen praktisch frei sind von nicht komplexierten toxisch wirkenden Metallionen.

Dies kann beispielsweise mit Hilfe von Farbindikatoren wie Xylenolorange durch Kontrolltitrationen während des Herstellungsprozesses gewährleistet werden. Die Erfindung betrifft daher auch Verfahren zur Herstellung der Komplexverbindungen und ihrer Salze. Als letzte Sicherheit bleibt eine Reinigung des isolierten Komplexsalzes.

Die erfindungsgemäßen pharmazeutischen Mittel enthalten vorzugsweise 1 µMol - 5 Mol/l des Komplexsalzes und werden in der Regel in Mengen von 0,001 - 20 mMol/kg dosiert. Sie sind zur enteralen und parenteralen Applikation bestimmt.

Die erfindungsgemäßen Komplexverbindungen kommen zur Anwendung
1. für die Röntgen- und NMR-Diagnostik in Form ihrer Komplexe mit den Ionen der Elemente mit den Ordnungszahlen 21 - 29, 42, 44 und 57 - 83;
2. für die Radiodiagnostik und Radiotherapie in Form ihrer Komplexe mit den Radioisotopen der Elemente mit den Ordnungszahlen 27, 29, 31, 37 - 39, 43, 49, 62, 64, 70, 75, 77 und 83.
3. für die Neutroneneinfangtherapie.

Die erfindungsgemäßen Mittel sind insbesondere hervorragend als Röntgenkontrastmittel geeignet, wobei besonders hervorzuheben ist, daß sich mit ihnen keine Anzeichen der von den iodhaltigen Kontrastmitteln bekannten anaphylaxieartigen Reaktionen in biochemisch-pharmakologischen Untersuchungen erkennen lassen. Die erfindungsgemäßen Substanzen erfüllen die vielfältigen Voraussetzungen, die an Kontrastmittel in der modernen Diagnostik zu stellen sind. Die Verbindungen und aus ihnen hergestellte Mittel zeichnen sich aus durch
- einen hohen Absorptionskoeffizienten für Röntgenstrahlen,
- eine gute Verträglichkeit, die notwendig ist, um den nichtinvasiven Charakter der Untersuchungen aufrechtzuerhalten,
- eine hohe Wirksamkeit, die notwendig ist, um den Körper mit möglichst geringen Mengen an Fremdstoffen zu belasten,
- eine gute Wasserlöslichkeit (Diese erlaubt es, hochkonzentrierte Lösungen herzustellen, wie sie besonders für die Verwendung als Röntgenkontrastmittel erforderlich sind womit die Volumenbelastung des Kreislaufs in vertretbaren Grenzen gehalten werden kann.),
- eine geringe Viskosität,
- eine geringe Osmolalität,
- eine günstige Ausscheidungskinetik.

Neben der überraschend hohen Wasserlöslichkeit der gegebenfalls paramagnetischen Schwermetallkomplexe, welche in einen für die Röntgendiagnostik erforderlichen Bereich gesteigert werden konnte, wirken sich die erfindungsgemäßen Verbindungen in der Röntgendiagnostik dadurch positiv aus, daß die erfindungsgemäßen Komplexverbindungen insbesondere auch Untersuchungen bei kurzwelligerer Röntgenstrahlung gestatten als dies mit konventionellen Kontrastmitteln möglich ist, wodurch die Strahlenbelastung des Patienten deutlich gemindert wird, da bekanntermaßen weiche Strahlung vom Gewebe sehr viel stärker absorbiert wird als harte [R. Felix, "Das Röntgenbild"; Thieme-Verlag Stuttgart (1980)].

Wegen der günstigen Absorptionseigenschaften der erfindungsgemäßen Kontrastmittel im Bereich harter Röntgenstrahlung sind die Mittel auch besonders für digitale Substraktionstechniken (die mit höheren Röhrenspannungen arbeiten) geeignet.

Details der Anwendung von Röntgenkontrastmitteln werden zum Beispiel in Barke, Röntgenkontrastmittel, G. Thieme, Leipzig (1970) und P. Thurn, E. Bücheler "Einführung in die Röntgendiagnostik", G. Thieme, Stuttgart, New York (1977) diskutiert.

Im allgemeinen werden die erfindungsgemäßen Mittel für die Anwendung als Röntgenkontrastmittel in Mengen von 0.1 - 20 mMol/kg Körpergewicht, vorzugsweise 0,25 - 5 mMol/kg Körpergewicht dosiert.

Die erfindungsgemäßen Mittel erfüllen, falls sie paramagnetisch sind, auch die vielfältigen Voraussetzungen für die Eignung als Kontrastmittel für die Kernspintomographie. So sind sie hervorragend dazu geeignet, nach oraler oder parenteraler Applikation durch Erhöhung der Signalintensität das mit Hilfe des Kernspintomographen erhaltene Bild in seiner Aussagekraft zu verbessern. Ferner zeigen sie die hohe Wirksamkeit, die notwendig ist, um den Körper mit möglichst geringen Mengen an Fremdstoffen zu belasten und die gute Verträglichkeit, die notwendig ist, um den nichtinvasiven Charakter der Untersuchungen aufrechtzuerhalten.

Im allgemeinen werden die erfindungsgemäßen Mittel für die Anwendung als NMR-Diagnostika in Mengen von 0,001 - 5 mMol/kg, vorzugsweise 0,005 - 0,5 mMol/kg, dosiert. Details der Anwendung werden zum Beispiel in H.J. Weinmann et al., Am. J. of Roentgenology 142, 619 (1984) diskutiert.

Besonders niedrige Dosierungen (unter 1 mg/kg Körpergewicht) von organspezifischen NMR-Diagnostika sind zum Beispiel zum Nachweis von Tumoren und von Herzinfarkt einsetzbar.

Ferner können die erfindungsgemäßen Komplexverbindungen vorteilhaft als Suszeptibilitäts-Reagenzien und als Shift-Reagenzien für die in-vivo NMR-Spektroskopie verwendet werden.

Sind die erfindungsgemäßen Mittel radioaktiv, so sind sie aufgrund ihrer günstigen Eigenschaften und der guten Stabilität der in ihnen enthaltenen Komplexverbindungen auch als Radio-diagnostika geeignet. Details ihrer Anwendung und Dosierung werden z.B. in "Radiotracers for Medical Applications", CRC-Press, Boca Raton, Florida, beschrieben.

Eine weitere bildgebende Methode mit Radioisotopen ist die Positronen-Emissions-Tomographie, die positronenemittierende Isotope wie z.B. ⁴³Sc, ⁴⁴Sc, ⁵²Fe, ⁵⁵Co und ⁶⁶Ga verwendet [Heiss, W.D.; Phelps, M.E.; Positron Emission Tomography of Brain, Springer-Verlag Berlin, Heidelberg, New York (1983)].

Die erfindungsgemäßen Verbindungen können auch in der Radioimmuno- oder Strahlentherapie verwendet werden. Diese unterscheidet sich von der entsprechenden Diagnostik nur durch die Menge und Art des verwendeten Isotops. Ziel ist dabei die Zerstörung von Tumorzellen durch energiereiche kurzwellige Strahlung mit einer möglichst geringen Reichweite. Geeignete β-emittierende Ionen sind z.B. ⁴⁶Sc, ⁴⁷Sc, ⁴⁸Sc, ⁷²Ga, ⁷³Ga und ⁹⁰Y. Geeignete geringe Halbwertszeiten aufweisende α-emittierende Ionen sind z.B. ²¹¹Bi, ²¹²Bi, ²¹³Bi, ²¹⁴Bi, wobei ²¹²Bi bevorzugt ist. Ein geeignetes Photonen- und Elektronen-emittierendes Ion ist ¹⁵⁸Gd, das aus ¹⁵⁷Gd durch Neutroneneinfang erhalten werden kann.

Ist das erfindungsgemäße Mittel zur Anwendung in der von R.L Mills et. al. [Nature Vol. 336, (1988), S. 787] vorgeschlagene Variante der Strahlentherapie bestimmt, so muß sich das Zentralion von einem Mößbauer-Isotop, wie beispielsweise ⁵⁷Fe oder ¹⁵¹Eu ableiten.

Details der Anwendung von Radiotherapeutika werden z.B. in R.W. Kozak et al., TIBTEC, Oktober 1986, 262 diskutiert.

Die Fluoreszenz-Eigenschaften insbesondere der erfindungsgemäßen Eu- und Tb-Komplexe können für die Nah-Infrarot-Bildgebung ausgenutzt werden.

Die Applikation der wäßrigen Röntgen- und NMR-Kontrastmittellösungen kann enteral oder parenteral, nämlich oral, rektal, intravenös, intraarteriell, intravasal, intracutan, subcutan (Lymphographie), subarachnoidal (Myelographie) erfolgen, wobei die intravenöse Applikation bevorzugt ist.

Die erfindungsgemäßen Mittel weisen nicht nur eine hohe Stabilität in vitro auf, sondern auch eine überraschend hohe Stabilität in vivo, so daß eine Freigabe oder ein Austausch der in den Komplexen nicht kovalent gebundenen - an sich giftigen - Ionen innerhalb der Zeit, in der die neuen Kontrastmittel vollständig wieder ausgeschieden werden, nicht erfolgt.

Insgesamt ist es gelungen, neue Komplexbildner, Metallkomplexe und Metallkomplexsalze zu synthetisieren, die neue Möglichkeiten in der diagnostischen und therapeutischen Medizin erschließen.

Die nachfolgenden Beispiele dienen zur näheren Erläuterung des Erfindungsgegenstandes.

### Beispiel 1

### Digadoliniumkomplex des Tetranatriumsalzes der N,N,N',N'-Tetrakis-{2-[N",N"-bis-(carboxymethyl)-amino]-ethyl}-meso-2,3-diaminobernsteinsäure

### a) N,N,N',N'-Tetrakis-{2-[N",N"-bis-((tert.-butyloxycarbonyl)-methyl)-amino]-ethyl}-meso-2,3-diaminobernsteinsäure-di-tert.-butylester

10,9 g (32,7 mmol) meso-2,3-Diaminobernsteinsäure-di-tert.-butylester-dihydrochlorid (Biernat, Rosc. Chem. 43 421 (1969)) und 51,8 g (147 mmol) N,N-Bis-[(tert.-butyloxycarbonyl)-methyl]-2-bromethylamin (M. Williams und H. Rapoport, J. Org. Chem. 58, 1151 (1993)) werden in 50 ml Acetonitril vorgelegt und mit 40 ml 2 n Phosphatpufferlösung (pH 8,0) versetzt. Der Ansatz wird bei Raumtemperatur 24 Stunden kräftig gerührt, wobei die wäßrige Phosphatpufferphase nach 2 und 8 Stunden gegen frische Pufferlösung ausgetauscht wird. Dann wird die organische Phase im Vakuum eingedampft und der Rückstand an Kieselgel mit Hexan/Essigsäureethylester/Triethylamin (3:1:0,01) chromatographiert. Die produkthaltigen Fraktionen werden im Vakuum eingedampft.
Ausbeute: 35,4 g (82,3 % d. Th.) farbloses Öl.

| Analyse (bezogen auf lösungsmittelfreie Substanz): | | | |
|---|---|---|---|
| ber. | C 60,69 | H 9,29 | N 6,24 |
| gef. | C 60,58 | H 9,44 | N 6,11 |

### b) N,N,N',N'-Tetrakis-{2-[N",N"-bis-(carboxymethyl)-amino]-ethyl}-meso-2,3-diaminobernsteinsäure

26,6 g (19,8 mmol) des in Beispiel 1a) beschriebenen Decaesters werden in 150 ml Methanol gelöst und mit 119 ml 2 n Natronlauge versetzt. Man kocht ca. 2 Stunden unter Rückfluß, zieht das Methanol im Vakuum ab und rührt weitere 3 Stunden bei 60°C. Anschließend stellt man mit konzentrierter Salzsäure pH 1 ein, dampft im Vakuum bis zur Trockne ein und rührt den Rückstand mit Isopropanol aus. Nach Filtrieren und Eindampfen des Filtrats im Vakuum erhält man ein farbloses Öl.
Ausbeute: 14,0 g (90,3 % d. Th.)

| Analyse (bezogen auf wasserfreie Substanz): | | | |
|---|---|---|---|
| ber. | C 42,86 | H 5,65 | N 10,71 |
| gef. | C 42,92 | H 5,81 | N 10,57 |

### c) Digadoliniumkomplex des Tetranatriumsalzes der N,N,N',N'-Tetrakis-{2-[N",N"-bis-(carboxymethyl)-amino]-ethyl}-meso-2,3-diaminobernsteinsäure

10,1 g (12,9 mmol) der in Beispiel 1b) beschriebenen Decasäure werden in 100 ml Wasser aufgenommen, mit 4,67g (12,9 mmol) Gadoliniumoxid versetzt und 3 Stunden bei 60°C gerührt. Anschließend stellt man mit verdünnter Natronlauge pH 7,2 ein, filtriert und gefriertrocknet das Filtrat.
Ausbeute: 14,7 g (96,7 % d. Th.) farbloses Lyophilisat

| Analyse (bezogen auf lösungsmittelfreie Substanz): | | | | | |
|---|---|---|---|---|---|
| ber. | C 28,47 | H 2,90 | Gd 26,63 | N 7,12 | Na 7,79 |
| gef. | C 28,64 | H 2,98 | Gd 26,53 | N 7,08 | Na 7,89 |

### Beispiel 2

### Digadoliniumkomplex des Tetranatriumsalzes der N,N,N',N'-Tetrakis-{2-[N",N"-bis-(carboxymethyl)-amino]-ethyl}-meso-2,6-diaminopimelinsäure

### a) N,N,N",N"-Tetrakis-{2-[N",N"-bis-((tert.-butyloxycarbonyl)-methyl)-amino]-ethyl}-meso-2,6-diaminopimelinsäure-di-tert.-butylester

11,3 g (30,1 mmol) meso-2,3-Diaminopimelinsäure-di-tert.-butylester-dihydrochlorid (Bricas et al., Bull. Soc. Chim. Fr. (1965) 1813) und 47,7 g (135 mmol) N,N-Bis-[(tert.-butyloxycarbonyl)-methyl]-2-bromethylamin (M. Williams und H. Rapoport, J. Org. Chem. 58, 1151 (1993)) werden in 50 ml Acetonitril vorgelegt und mit 40 ml 2 n Phosphatpufferlösung (pH 8,0) versetzt. Der Ansatz wird bei Raumtemperatur 24 Stunden kräftig gerührt, wobei die wäßrige Phosphatpufferphase nach 2 und 8 Stunden gegen frische Pufferlösung ausgetauscht wird. Dann wird die organische Phase im Vakuum eingedampft und der Rückstand an Kieselgel mit Hexan/Essigsäureethylester/Triethylamin (3:1:0,01) chromatographiert. Die produkthaltigen Fraktionen werden im Vakuum eingedampft.
Ausbeute: 33,7 g (80,6 % d. Th.) farbloses Öl.

| Analyse (bezogen auf lösungsmittelfreie Substanz): | | | |
|---|---|---|---|
| ber. | C 61,45 | H 9,44 | N 6,06 |
| gef. | C 61,54 | H 9,63 | N 5,92 |

### b) N,N,N',N'-Tetrakis-{2-[N",N"-bis-(carboxymethyl)-amino]-ethyl}-meso-2,3-diaminopimelinsäure

30,6 g (22,0 mmol) des in Beispiel 2a) beschriebenen Decaesters werden in 150 ml Methanol gelöst und mit 132 ml 2 n Natronlauge versetzt. Man kocht ca. 2 Stunden unter Rückfluß, zieht das Methanol im Vakuum ab und rührt weitere 3 Stunden bei 60°C. Anschließend stellt man mit konzentrierter Salzsäure pH 1 ein, dampft im Vakuum bis zur Trockne ein und rührt den Rückstand mit Isopropanol aus. Nach Filtrieren und Eindampfen des Filtrats im Vakuum erhält man ein farbloses Öl.
Ausbeute: 17,3 g (94,8.% d Th.)

| Analyse (bezogen auf salzsäurefreie Substanz): | | | |
|---|---|---|---|
| ber. | C 45,04 | H 6,10 | N 10,17 |
| gef. | C 44,95 | H 5,92 | N 10,20 |

### c) Digadoliniumkomplex des Tetranatriumsalzes der N,N,N',N'-Tetrakis-{2-[N",N"-bis-(carboxymethyl)-amino]ethyl}-meso-2,6-diaminopimelinsäure

15,4 g (18,6 mmol) der in Beispiel 2b) beschriebenen Decasäure werden in 100 ml Wasser aufgenommen, mit 6,75 g (18,6 mmol) Gadoliniumoxid versetzt und 3 Stunden bei 60°C gerührt. Anschließend stellt man mit verdünnter Natronlauge pH 7,2 ein, filtriert und gefriertrocknet das Filtrat.
Ausbeute: 20,3 g (89,3 % d. Th.) farbloses Lyophilisat

| Analyse (bezogen auf lösungsmittelfreie Substanz): | | | | | |
|---|---|---|---|---|---|
| ber. | C 30,44 | H 3,30 | Gd 25,71 | N 6,87 | Na 7,52 |
| gef. | C 30,41 | H 3,41 | Gd 25,66 | N 6,93 | Na 7,48 |

### Beispiel 3

### Diytterbiumkomplex des Tetranatriumsalzes der 3-{4-[2-[N,N-bis-[2-[N',N'-bis-(carboxymethyl)-amino]ethyl]]-amino-2-carboxyethyl]-phenoxy}-2-N-{2-[N'-[2-[N",N"-bis-(carboxymethyl)-amino]-ethyl]-N'-(carboxymethyl)-amino]-ethyl}-N(carboxymethyl)-amino}-propionsäure

### a) 3-{4-[2-[N,N-bis-[2-N',N'-bis-(benzyloxycarbonylmethyl)-amino]-ethyl]]-amino-2-(benzyloxycarbonyl)-ethyl)]-phenoxy}-2-{N-(2-[N"-[2-[N",N"-bis-(tert.-butoxycarbonylmethyl)-amino]-ethyl]]-N'-(tert.-butoxycarbonylmethyl)-amino]-ethyl}-N(tert.-butoxy-carbonylmethyl)-amino}-propionsäure-tert.-butylester

10,15 g (10,7 mmol) der nach Beispiel 5a) herstellten Verbindung werden in 50 ml wasserfreiem N,N-Dimethylformamid gelöst und bei 0°C unter Argon mit 0,47 g (11,75 mmol) Natriumhydridsuspension (60 % in Öl) versetzt. Man läßt den Ansatz 15 Minuten rühren und gibt dann 9,18 g (10,7 mmol) des Tosylates aus Beispiel 7 c) zu. Wenn das Reaktionsgemisch Raumtemperatur erreicht hat, läßt man es noch 7 Stunden weiterrühren. Zur Aufarbeitung wird der Ansatz in Toluol aufgenommen und mehrmals gegen wässrige Natriumhydrogencarbonatlösung ausgeschüttelt. Die organische Phase wird abgetrennt, über Magnesiumsulfat getrocknet, filtriert und eingedampft. Der ölige Rückstand wird an Kieselgel mit Hexan/Diethylether/Triethylamin (30:80:1) chromatographiert, die produkthaltigen Fraktionen werden vereint und eingedampft.
Ausbeute: 12,8 g (73,1 % d. Th.) blaßgelbes Öl.

| Analyse (unter Berücksichtigung des Lösemittelgehaltes): | | | |
|---|---|---|---|
| ber. | C 66,81 | H 7,52 | N 5,14 |
| gef. | C 66,68 | H 7,70 | N 5,21 |

### b) 3-{4-[2-[(N,N-bis-[2-[N',N'-bis(carboxymethyl)-amino]-ethyl]]-amino-2-carboxyethyl]-phenoxy}-2-{N-(2-[N"-[2-[N",N"-bis(carboxymethyl)-amino]-ethyl]-N'(carboxymethyl)-amino]-ethyl}-N-(carboxymethyl)-amino}-propionsäure

12,5 g (7,6 mmol) des in Beispiel 3 a) beschriebenen Decaesters werden in 110 ml Methanol gelöst und mit 105 ml 2 n Natronlauge versetzt. Man kocht ca. 3,5 Stunden unter Rückfluß, zieht das Methanol im Vakuum ab und rührt weitere 4 Stunden bei 60°C. Anschließend stellt man mit konzentrierter Salzsäure pH 1 ein, dampft im Vakuum bis zur Trockne ein und rührt den Rückstand mit Ethanol aus. Nach Filtrieren und Eindampfen des Filtrats im Vakuum erhält man einen farblosen Feststoff.
Ausbeute: 6,2 g (90 % d. Th.).

| Analyse (unter Berücksichtigung des Lösemittelgehaltes): | | | |
|---|---|---|---|
| ber. | C 47,79 | H 5,79 | N 9,29 |
| gef. | C 47,87 | H 5,80 | N 9,14 |

### c) Diytterbiumkomplex des Tetranatriumsalzes der 3-{4-[2-[N,N-bis-[2-[N',N'-bis-(carboxymethyl)-amino]-ethyl]]-amino-2-carboxyethyl]-phenoxy}-2-(N-(2-[N'-[2-N",N"-bis(carboxymethyl)-amino]-ethyl]-N'-(carboxymethyl)-amino]-ethyl}-N-(carboxymethyl)-amino}-propionsäure

5,9 g (6,5 mmol) der Decasäure aus Beispiel 3b) werden in 90 ml Wasser aufgenommen, mit 1,28 g (3,25 mmol) Ytterbiumoxid versetzt und 8 Stunden bei 65°C gerührt. Anschließend stellt man mit verdünnter Natronlauge pH 7,2 ein, filtriert und gefriertrocknet das Filtrat.
Ausbeute: 7,9 g (91,2 % d. Th.) farbloses Lyophilisat.

| Analyse (bezogen auf lösungsmittelfreie Substanz): | | | | | |
|---|---|---|---|---|---|
| ber. | C 32,44 | H 3,18 | Yb 25,97 | N 6,31 | Na 6,90 |
| gef. | C 32,28 | H 3,40 | Yb 25,78 | N 6,21 | Na 6,73 |

### Beispiel 4

### Digadoliniumkomplex des Dinatriumsalzes des N,N,N',N'-Tetrakis-{2-[N",N"-bis-(carboxymethyl)-amino]-ethyl}-meso-2,3-diaminobernsteinsäure-N"',N""-bis-(2-methoxyethyl)-diamid

### a) meso-2,3-Diaminobernsteinsäure-N,N'-bis(2-methoxyethyl)-diamid, Diacetat

Eine Suspension von 13,9 g (50,1 mmol) meso-2,3-Diaminobernsteinsäurediethylester-Dihydrochlorid (Tamura, J. Biochem. Tokyo 27 [1938] 339) in 50 ml Ethanol wird mit 38,7g (0,51 mmol) 2-Methoxyethylamin versetzt und 12 Stunden unter Rückfluß gekocht. Anschließend wird vollständig eingedampft und der Rückstand an Kieselgel mit Dichlormethan/Methanol/Essigsäure chromatographiert. Nach dem Eindampfen der produkthaltigen Fraktionen erhält man ein leicht gelbliches Öl.
Ausbeute: 17,2 g (88,2 % d. Th.)

| Analyse: | | | |
|---|---|---|---|
| ber. | C 43,97 | H 7,91 | N 14,96 |
| gef. | C 44.04 | H 8,01 | N 14,83 |

### b) N,N,N',N'-Tetrakis-{2-[N",N"-bis-((benzyloxycarbonyl)-methyl)-amino]-ethyl}-meso-2,3-diaminobernsteinsäure-N"',N""-bis(2-methoxyethyl)-diamid

14,2 g (37,1 mmol) der nach Beispiel 4a) hergestellten Verbindung und 70,2 g (167 mmol) N,N-Bis-[(benzyloxycarbonyl)-methyl]-2-bromethylamin (M. Williams und H. Rapoport, J. Org. Chem. 58, 1151 (1993)) werden in 70 ml Acetonitril vorgelegt und mit 60 ml 2 n Phosphatpufferlösung (pH 8,0) versetzt. Der Ansatz wird bei Raumtemperatur 24 Stunden kräftig gerührt, wobei die wäßrige Phosphatpufferphase nach 2 und 8 Stunden gegen frische Pufferlösung ausgetauscht wird. Dann wird die organische Phase im Vakuum eingedampft und der Rückstand an Kieselgel mit Hexan/Essigsäureethylester/Triethylamin (3:1:0,01) chromatographiert. Die produkthaltigen Fraktionen werden im Vakuum eingedampft.
Ausbeute: 46,6 g (77,3 % d. Th.) farbloses Öl.

| Analyse (bezogen auf lösungsmittelfreie Substanz): | | | |
|---|---|---|---|
| ber. | C 66,73 | H 6,60 | N 6,92 |
| gef. | C 66,59 | H 6,65 | N 6,87 |

### c) N,N,N',N'-Tetrakis-{2-[N",N"-bis-(carboxymethyl)-amino]-ethyl}-meso-2,3-diaminobernsteinsäure-N"',N""-bis(2-methoxyethyl)-diamid

33,6 g (20,7 mmol) des in Beispiel 4b) beschriebenen Decaesters werden in 150 ml Methanol gelöst und mit 104 ml 2 n Natronlauge versetzt. Man kocht ca. 2 Stunden unter Rückfluß, zieht das Methanol im Vakuum ab und rührt weitere 3 Stunden bei 60°C. Anschließend stellt man mit konzentrierter Salzsäure pH 1 ein, dampft im Vakuum bis zur Trockne ein und rührt den Rückstand mit Isopropanol aus. Nach Filtrieren und Eindampfen des Filtrats im Vakuum erhält man ein farbloses Öl.
Ausbeute: 17,4 g (93,4 % d Th.)

| Analyse (bezogen auf salzsäurefreie Substanz): | | | |
|---|---|---|---|
| ber. | C 45,43 | H 6,50 | N 12,47 |
| gef. | C 45,35 | H 6,61 | N 12,34 |

### d) Digadoliniumkomplex des Dinatriumsalzes des N,N,N',N'-Tetrakis-{2-[N",N"-bis-(carboxymethyl)-amino]-ethyl}-meso-2,3-diaminobernsteinsäure-N"',N'"'-bis-(2-methoxyethyl)-diamid

16,8 g (18,7 mmol) der in Beispiel 4c) beschriebenen Octasäure werden in 150 ml Wasser aufgenommen, mit 6,78 g (18,7 mmol) Gadoliniumoxid versetzt und 3 Stunden bei 80°C gerührt. Anschließend stellt man mit verdünnter Natronlauge pH 7,2 ein, filtriert und gefriertrocknet das Filtrat.
Ausbeute: 22,3 g (95,3 % d. Th.) farbloses Lyophilisat

| Analyse (bezogen auf lösungsmittelfreie Substanz): | | | | | |
|---|---|---|---|---|---|
| ber. | C 32,64 | H 4,03 | N 8,95 | Gd 25,13 | Na 3,68 |
| gef. | C 32,65 | H 4,09 | N 9,04 | Gd 25,06 | Na 3,59 |

### Beispiel 5

### Digadoliniumkomplex des Tetranatriumsalzes von 1,8-Bis-{4-[2(N,N-bis-(2-(N',N'-bis-(carboxymethyl)-amino)-ethyl)-amino)-2-carboxyethyl]-phenoxy}-octan

### a) N,N-Bis-{2-[N',N'-bis-[(benzyloxycarbonyl)-methyl]-amino]-ethyl}-L-tyrosinbenzylester

15,5 g (35,0 mmol) L-Tyrosinbenzylester-4-methylbenzolsulfonat und 66,2 g (158 mmol) N,N-Bis-[(benzyloxycarbonyl)-methyl]-2-bromethylamin (M. Williams und H. Rapoport, J. Org. Chem. 58, 1151 (1993)) werden in 70 ml Acetonitril vorgelegt und mit 60 ml 2 n Phosphatpufferlösung (pH 8,0) versetzt. Der Ansatz wird bei Raumtemperatur 24 Stunden kräftig gerührt, wobei die wäßrige Phosphatpufferphase nach 2 und 8 Stunden gegen frische Pufferlösung ausgetauscht wird. Dann wird die organische Phase im Vakuum eingedampft und der Rückstand an Kieselgel mit Hexan/Essigsäureethylester/Triethylamin (3:1:0,01) chromatographiert. Die produkthaltigen Fraktionen werden im Vakuum eingedampft.
Ausbeute: 23.4 g (70,3 % d. Th.) farbloses Öl.

| Analyse (bezogen auf lösungsmittelfreie Substanz): | | | |
|---|---|---|---|
| ber. | C 70,79 | H 6,26 | N 4,42 |
| gef. | C 70,69 | H 6,33 | N 4,51 |

### b) 1,8-Bis-{4-[2-(N,N-bis(2-(N',N'-bis((benzyloxycarbonyl)-methyl)-amino)-ethyl)-amino)-2(benzyloxycarbonyl)-ethyl]-phenoxy}-octan

20,3 g (21,4 mmol) der nach Beispiel 5a) herstellten Verbindung werden in 50 ml wasserfreiem N,N-Dimethylformamid gelöst und bei 0°C unter Argon mit 0,94 g (23,5 mmol) Natriumhydridsuspension (60 % in Öl) versetzt. Man läßt den Ansatz 15 Minuten rühren und gibt dann 3,91 g (10,7 mmol) 1,8-Diiodoctan zu. Wenn das Reaktionsgemisch Raum-temperatur erreicht hat, läßt man es noch 5 Stunden weiterrühren. Zur Aufarbeitung wird der Ansatz in Toluol aufgenommen und mehrmals gegen wässrige Natriumhydrogencarbonatlösung ausgeschüttelt. Die organische Phase wird abgetrennt, über Magnesiumsulfat getrocknet, filtriert und eingedampft. Der ölige Rückstand wird an Kieselgel mit Hexan/Diethylether/Triethylamin (30:80:1) chromatographiert, die produkthaltigen Fraktionen werden vereint und eingedampft.
Ausbeute: 15,3 g (71,1 % d. Th.) farbloses Öl

| Analyse (bezogen auf lösungsmittelfreie Substanz): | | | |
|---|---|---|---|
| ber. | C 71,69 | H 6,62 | N 4,18 |
| gef. | C 71,53 | H 6,78 | N 4,07 |

### c) 1,8-Bis-{4-[2-(N,N-bis-(2-(N',N'-bis-(carboxymethyl)-amino)-ethyl)-amino)-2-carboxyethyl]-phenoxy}-octan

14,5 g (7,21 mmol) des in Beispiel 5b) beschriebenen Decaesters werden in 145 ml Methanol gelöst und nach Zugabe von 1,4 g Palladium (10 %) auf Aktivkohle unter Wasserstoffatmosphäre bis zur beendeten Wasserstoffaufnahme hydriert. Nach Filtrieren und Eindampfen des Filtrats im Vakuum erhält man ein farbloses Öl.
Ausbeute: 7,95 g (99,4% d Th.)

| Analyse (bezogen auf lösungsmittelfreie Substanz): | | | |
|---|---|---|---|
| ber. | C 54,15 | H 6,54 | N 7,58 |
| gef. | C 54,00 | H 6,62 | N 7,47 |

### d) Digadoliniumkomplex des Tetranatriumsalzes von 1,8-Bis-{4-[2-(N,N-bis-(2(N',N'-bis-(carboxymethyl)-amino)-ethyl)-amino)-2-carboxyethyl]-phenoxy}-octan

7,87 g (7,10 mmol) der in Beispiel 5c) beschriebenen Decasäure werden in 25 ml Wasser aufgenommen, mit 2,57 g (7,10 mmol) Gadoliniumoxid versetzt und 3 Stunden bei 60°C gerührt. Anschließend stellt man mit verdünnter Natronlauge pH 7,2 ein, filtriert und gefriertrocknet das Filtrat.
Ausbeute: 10,5 g (98,6 % d. Th.) farbloses Lyophilisat

| Analyse (bezogen auf lösungsmittelfreie Substanz): | | | | | |
|---|---|---|---|---|---|
| ber. | C 39,89 | H 4,15 | Gd 20,89 | N 5,58 | Na 6,11 |
| gef. | C 39,88 | H 4,23 | Gd 20,78 | N 5,62 | Na 6,18 |

### Beispiel 6

### Didysprosiumkomplex des Tetranatriumsalzes der N,N'-bis-{2-[N",N"-bis(carboxymethyl)-amino]-3-[(4-methoxy)-phenyl]-propyl}-N,N'-bis-{2-[N"',N"'-bis-(carboxymethyl)-amino]-ethyl}-meso-2,3-diaminobernsteinsäure

### a) N,N'-Dibenzyl-meso-2,3-diaminobernsteinsäure-di-tert.-butylester

11,9 g (35,7 mmol) meso-2,3-Diaminobernsteinsäure-di-tert.-butylester-dihydrochlorid (Biernat, Rosc. Chem. 43, 421 (1969)) und 8,33 g (78,6 mmol) Benzaldehyd werden in 50ml Methanol 3 Stunden bei 24°C gerührt und anschließend mit 3,37 g (53,6 mmol) Natriumcyanoborhydrid versetzt. Nach 48 Stunden Rühren bei Raumtemperatur wird der Ansatz durch vorsichtige Zugabe von halbkonzentrierter Salzsäure auf pH 2 eingestellt, dann mit konzentrierter wäßriger Natriumhydrogencarbonatlösung neutralisiert und nach weitgehendem Abdampfen von Methanol mit Essigsäureethylester ausgeschüttelt. Die organische Phase wird über wasserfreiem Magnesiumsulfat getrocknet, filtriert und eingedampft. Der Rückstand wird an Kieselgel mit Diethylether/Hexan/Triethylamin (70:30:1) chromatographiert; die produkthaltigen Fraktionen werden vereinigt und eingedampft.
Ausbeute: 9,69 g (61,1 % d. Th.) farbloses Öl

| Analyse (bezogen auf lösungsmittelfreie Substanz): | | | |
|---|---|---|---|
| ber. | C 70,88 | H 8,24 | N 6,36 |
| gef. | C 70,72 | H 8,33 | N 6,41 |

### b) N,N'-Dibenzyl-N,N',-bis-{2-[N",N"-bis((tert.-butyloxycarbonyl)-methyl)-amino]-ethyl}-meso-2,3-diaminobernsteinsäure-di-tert.-butylester

9,21 g (20,9 mmol) der nach Beispiel 6a) hergestellten Verbindung und 10,2 g (52,3 mmol) N,N-Bis-[(tert.-butyloxycarbonyl)-methyl]-2-bromethylamin (M. Williams und H. Rapoport, J. Org. Chem. 58, 1151 (1993)) werden in 30 ml Acetonitril vorgelegt und mit 20 ml 2 n Phosphatpufferlösung (pH 8,0) versetzt. Der Ansatz wird bei Raumtemperatur 24 Stunden kräftig gerührt, wobei die wäßrige Phosphatpufferphase nach 2 und 8 Stunden gegen frische Pufferlösung ausgetauscht wird. Dann wird die organische Phase im Vakuum eingedampft und der Rückstand an Kieselgel mit Hexan/Essigsäureethylester/Triethylamin (3:1:0,01) chromatographiert. Die produkthaltigen Fraktionen werden im Vakuum eingedampft.
Ausbeute: 16,2 g (78,9 % d. Th.) farbloses Öl

| Analyse (bezogen auf lösungsmittelfreie Substanz): | | | |
|---|---|---|---|
| ber. | C 65,96 | H 8,82 | N 5,70 |
| gef. | C 66,07 | H 8,74 | N 5,77 |

### c) N,N",bis-{2-[N",N"-bis((tert.-butyloxycarbonyl)-methyl)-amino]-ethyl}-meso-2,3-diaminobernsteinsäure-di-tert.-butylester

15,3 g (15,6 mmol) der nach Beispiel 6b) hergestellten Verbindung werden in 75 ml Ethanol gelöst und nach Zugabe von 1,5 g Palladium (10 %) auf Aktivkohle unter Wasserstoffatmosphäre bei Raumtemperatur bis zur beendeten Wasserstoffaufnahme hydriert. Nach Filtrieren und Eindampfen des Filtrats im Vakuum erhält man ein farbloses Öl.
Ausbeute: 12,5 g (100 % d. Th.)

| Analyse: | | | |
|---|---|---|---|
| ber. | C 59,83 | H 9,29 | N 6,98 |
| gef. | C 59,96 | H 9,15 | N 6,88 |

### d) 2-[N,N-bis((tert.-butyloxycarbonyl)-methyl)-amino]-3-[(4-methoxy)-phenyl]-propanol

18,1 g (100 mmol) 2-Amino-3-[(4-methoxy)-phenyl]-propanol (L. Berlinguet, Can. J. Chem. **32,** 31 (1954)) werden in 100 ml Tetrahydrofuran gelöst und mit 10 ml Wasser und 20,7 g (150 mmol) Kaliumcarbonat versetzt. Nach Zutropfen von 42,9 g (220 mmol) Bromessigsäure-tert.-butylester wird 3 Tage bei 60°C gerührt. Nach dem Abkühlen wird filtriert, im Vakuum eingeengt und der Rückstand an Kieselgel mit Diethylether/Hexan/Triethylamin (70:20:5) chromatographiert. Die Produktfraktionen werden im Vakuum eingedampft und getrocknet.
Ausbeute: 36,7 g (89,6 % d. Th.) farbloses Öl

| Analyse (bezogen auf lösungsmittelfreie Substanz): | | | |
|---|---|---|---|
| ber. | C 64,52 | H 8,61 | N 3,42 |
| gef. | C 64,55 | H 8.50 | N 3,51 |

### e) 1-Brom-2-[N,N-bis-((tert.-butyloxycarbonyl)-methyl)-amino]-3-[(4-methoxy)-phenyl]-propan

Eine Lösung von 35,8 g (75,8 mmol) der nach Beispiel 6d erhaltenen Verbindung und 22,9 g (87,1 mmol) Triphenylphosphin in 400 ml Dichlormethan wird bei 0°C portionsweise mit 15,5 g (87,1 mmol) N-Bromsuccinimid versetzt und anschließend über Nacht bei Raumtemperatur gerührt. Die Lösung wird eingedampft und der Rückstand mit tert.-Butyl-methylether verrieben. Es entsteht ein Niederschlag, der abgetrennt und mit tert.-Butyl-methylether gewaschen wird. Die vereinigten Filtrate werden eingedampft und der Rückstand an Kieselgel mit Hexan/Diethylether (2:1) chromatographiert. Eindampfen der Produktfraktionen ergibt ein farbloses Öl.
Ausbeute: 32,6 g (91,0 % d. Th.)

| Analyse: | | | | |
|---|---|---|---|---|
| ber. | C 55,93 | H 7,25 | Br 16,91 | N 2,97 |
| gef. | C 56,12 | H 7,26 | Br 16,97 | N 2,83 |

### f) N,N'-bis-{2-[N",N"-bis((tert.-butyloxycarbonyl)-methyl)-amino]-3-[(4-methoxy)-phenyl]-propyl}-N,N'-bis-{2-[N"',N'''-bis((tert.-butyloxycarbonyl)-methyl)-amino]-ethyl}-meso-2,3-diaminobernsteinsäure-di-tert.-butylester

8,34 g (10,4 mmol) der nach Beispiel 6c) und 10,8 g (22,8 mmol) der nach Beispiel 6e) hergestellten Verbindung werden in 20 ml Acetonitril vorgelegt und mit 15 ml 2 n Phosphatpufferlösung (pH 8,0) versetzt. Der Ansatz wird bei Raumtemperatur 24 Stunden kräftig gerührt, wobei die wäßrige Phosphatpufferphase nach 2 und 8 Stunden gegen frische Pufferlösung ausgetauscht wird. Dann wird die organische Phase im Vakuum eingedampft und der Rückstand an Kieselgel mit Hexan/Essigsäureethylester/Triethylamin (3:1:0,01) chromatographiert. Die produkthaltigen Fraktionen werden im Vakuum eingedampft.
Ausbeute: 10,8 g (65,6 % d. Th.) farbloses Öl

| Analyse (bezogen auf lösungsmittelfreie Substanz): | | | |
|---|---|---|---|
| ber. | C 51,56 | H 5,90 | N 8,20 |
| gef. | C 51,49 | H 6,01 | N 8,33 |

### g) N,N'-bis-{2-[N',N'-bis-(carboxymethyl)-amino]-3-[(4-methoxy)-phenyl]-propyl}-N,N'-bis-{2-[N"',N""-bis-(carboxymethyl)-amino]-ethyl}-meso-2,3-diaminobernsteinsäure

10,1 g (6,36 mmol) der nach Beispiel 6f) hergestellten Verbindung werden in 20 ml Methanol gelöst und unter Rühren tropfenweise mit 80 ml halbkonzentrierter Salzsäure versetzt. Nach 3 Stunden Rühren bei Raumtemperatur wird im Vakuum eingedampft und getrocknet.
Ausbeute: 6,53 g (100 % d. Th.) farbloses Öl

| Analyse (bezogen auf salzsäurefreie Substanz): | | | |
|---|---|---|---|
| ber. | C 51,56 | H 5,90 | N 8,20 |
| gef. | C 51,67 | H 5,95 | N 8,11 |

### h) Didysprosiumkomplex des Tetranatriumsalzes der N,N'-bis-{2-[N",N"-bis-(carboxymethyl)-amino]-3-[(4-methoxy)-phenyl]-propyl}-N,N'-bis-{2-[N"',N"'-bis-(carboxymethyl)-amino]-ethyl}-meso-2,3-diaminobernsteinsäure

5,95 g (5,80 mmol) der in Beispiel 6g) beschriebenen Decasäure werden in 20 ml Wasser aufgenommen, mit 2,17 g (5,80 mmol) Dysprosiumoxid versetzt und 3 Stunden bei 60°C gerührt. Anschließend stellt man mit verdünnter Natronlauge pH 7,2 ein, filtriert und gefriertrocknet das Filtrat.
Ausbeute: 7,76 g (93,4 % d. Th.) farbloses Lyophilisat

| Analyse (bezogen auf lösungsmittelfreie Substanz): | | | | | |
|---|---|---|---|---|---|
| ber. | C 36,91 | H 3,52 | Dy 22,70 | N 5,87 | Na 6,42 |
| gef. | C 37,10 | H 3,55 | Dy 22,59 | N 5,92 | Na 6,38 |

### Beispiel 7

### Digadoliniumkomplex des Tetranatriumsalzes der 3,6,9,15,18,21-Hexaaza-10,14-bis-carboxy-3,6,9,15,18,21-hexakis(carboxymethyl)-12-oxa-tricosan-1,23-disäure

### a) 3-(Phenylmethoxy)-2{-N-(2-[N'-[2-[N",N"-bis(tert.-butyloxycarbonylmethyl)-amino]-ethyl]]-N'-(tert.-butyloxycarbonylmethyl)-amino]-ethyl}-N-(tert.-butyloxycarbonylmethyl)-amino}-propionsäure

5,13 g (10 mmol) 3-Phenylmethoxy-2-N-{2'-N'-[2"-N",N"-bis-(carboxymethyl)-aminoethyl]-N'-(carboxymethyl)-aminoethyl]-N'-(carboxymethyl)-aminopropionsäure (Beispiel 4B der EP 230 893) werden in 60 ml tert.-Butylacetat mit 0,8 ml (9 mmol) Perchlorsäure (70 %) bei Raumtemperatur versetzt und 3 Tage gerührt. Nach beendeter Reaktion wird der Ansatz auf Eis/Wasser gegossen und die Säure neutralisiert. Man extrahiert mehrmals mit Diethylether, trocknet die organische Phase über Natriumsulfat, filtriert und dampft den Ether ab. Der Rückstand wird über Kieselgel chromatographiert.
Ausbeute: 5,4 g (68 % d. Th.) farbloses Öl.

| Analyse (unter Berücksichtigung des Lösemittelgehaltes): | | | |
|---|---|---|---|
| ber. | C 63,53 | H 9,01 | N 5,29 |
| gef. | C 63,29 | H 9,24 | N 5,24 |

### b) 3-Hydroxy-2-{N-{2-[N'-[2-[N",N"-bis-(tert.-butyloxycarbonylmethyl)-amino]-ethyl]-N'-(tert.-butyloxycarbonylmethyl)-amino]-ethyl}-N-(tert.-butyloxycarbonylmethyl)-amino}-propionsäure

4,95 g (6,2 mmol) des Benzylethers aus Beispiel 7 a) werden in 30 ml Methanol gelöst und nach Zugabe von 1,4 g Palladium (10 %) auf Aktivkohle unter Wasserstoffatmosphäre bei Raumtemperatur bis zur beendeten Wasserstoffaufnahme hydriert. Nach Filtrieren und Eindampfen des Filtrats im Vakuum erhält man ein farbloses Öl.
Ausbeute: 4,1 g (94 % d. Th.).

| Analyse (unter Berücksichtigung des Lösemittelgehaltes): | | | |
|---|---|---|---|
| ber. | C 59,72 | H 9,31 | N 5,97 |
| gef. | C 59,65 | H 9,14 | N 6,11 |

### c) 3-(4-Methylbenzolsulfonyloxy)-2-{N-{2-[N'-[2-[N",N"-bis-(tert.-butyloxycarbonylmethyl)-amino]-ethyl]-N'-(tert.-butyloxycarbonylmethyl)-amino]-ethyl]-N-(tert.-butyloxycarbonylmethyl)-amino }propionsäure

3,8 g (5,4 mmol) des Alkohols aus Beispiel 7 b) werden in 40 ml Dichlormethan und 0,84 ml (6,5 mmol) Triethylamin bei 0°C unter Stickstoff gerührt und tropfenweise mit 1,13 g (6 mmol) Methansulfonsäurechlorid versetzt. Man läßt die Reaktionstemperatur innerhalb von 3 Stunden auf Raumtemperatur steigen, und schüttelt den Ansatz mit gesättigter Natriumhydrogencarbonatlösung aus. Die organische Phase wird über Natriumsulfat getrocknet, filtriert und eingedampft.
Ausbeute: 3,5 g (75,5 % d. Th.) schwach-gelbliches Öl

| Analyse (bezogen auf lösungsmittelfreie Substanz): | | | | |
|---|---|---|---|---|
| ber. | C 58,79 | H 8,34 | N 4,90 | S 3,74 |
| gef. | C 58,63 | H 8,51 | N 4,73 | S 3,60 |

### d) 3,6,9,15,18,21-Hexaaza-10,14-bis-(tert.-butyloxycarbonyl)-3,6,9,15,18,21-hexakis(tert.-butyloxycarbonylmethyl)-12-oxa-tricosan-1,23-disäure-di-tert.-butylester

Eine Lösung von 5,36 g (7,61 mmol) des nach Beispiel 7 b) hergestellten Alkohols in 20 ml wasserfreiem N,N-Dimethylformamid wird bei 0°C mit 0,33 g (8,25 mmol) Natriumhydrid (60 % in Öl) versetzt und unter Argon 15 Minuten gerührt. Dann gibt man 6,53 g (7,61 mmol) des nach Beispiel 7 c) hergestellten Tosylats und 0,13 g (0,76 mmol) Kaliumiodid zu und rührt das Reaktionsgemisch eine Stunde bei 0°C und acht Stunden bei Raumtemperatur. Zur Aufarbeitung wird das Reaktionsgemisch in 100 ml Essigsäureethylester aufgenommen und gegen wäßrige Natriumhydrogencarbonatlösung ausgeschüttelt. Die organische Phase wird über Natriumsulfat getrocknet, filtriert und eingedampft. Der Rückstand wird an Kieselgel chromatographiert und die produkthaltigen Fraktionen vereinigt und im Vakuum eingedampft.
Ausbeute: 6,7 g (63,3 % d. Th.) farbloses Öl.

| Elementaranalyse (unter Berücksichtigung des Lösemittelgehaltes): | | | |
|---|---|---|---|
| ber. | C 60,50 | H 9,28 | N 6,05 |
| gef. | C 60,38 | H 9,33 | N 6,21 |

### e) Digadoliniumkomplex des Tetranatriumsalzes der 3,6,9,15,18,21-Hexaaza-10,14-bis-carboxy-3,6,9,15,18,21-hexakis-(carboxymethyl)-12-oxa-tricosan-1,23-disäure

6,65 g (4,8 mmol) des in Beispiel 7d) beschriebenen Decaesters werden in 40 ml Methanol gelöst und mit 30 ml 2 n Natronlauge versetzt. Man kocht ca. 3 Stunden unter Rückfluß, zieht das Methanol im Vakuum ab und rührt weitere 3 Stunden bei 60°C. Anschließend stellt man mit konzentrierter Salzsäure pH 1 ein, dampft im Vakuum bis zur Trockne ein und rührt den Rückstand mit Ethanol aus. Nach Filtrieren und Eindampfen des Filtrats im Vakuum erhält man einen farblosen Feststoff, den man in Wasser suspendiert und nach Beispiel 1c) den Liganden mit Gadoliniumoxid komplexiert. Nach erfolgter Komplexierung stellt man mit 2 n Natronlauge den pH-Wert auf 7,3 ein und lyophilisiert die wäßrige Lösung.
Ausbeute: 5,2 g (88,4 % d. Th.) farbloses Lyophilisat.

| Analyse (bezogen auf lösungsmittelfreie Substanz): | | | | | |
|---|---|---|---|---|---|
| ber. | C 29,41 | H 3,13 | Gd 25,67 | N 6,86 | Na 7,51 |
| gef. | C 29,34 | H 3,41 | Gd 25,59 | N 6,64 | Na 7,24 |

### Beispiel 8

### Didysprosiumkomplex der N,N,N',N'-Tetrakis-{2-[[N"-(carboxymethyl)]-N"((N''-methyl)-carbamoylmethyl)-amino]-ethyl}meso-2,6-diaminopimelinsäure

### a) N,N,N',N'-Tetrakis-[2-(2,6-dioxomorpholino)-ethyl]-meso-2,6-diaminopimelinsäure

8,27 g (10,0 mmol) der nach Beispiel 1b) hergestellten Decasäure werden in 50 ml Essigsäureanhydrid unter Feuchtigkeitsausschluß 5 Stunden unter Rückfluß gekocht. Im Anschluß wird vollständig eingedampft und der Rückstand am Ölpumpenvakuum getrocknet.
Ausbeute: 75,5 g (100 % d. Th) farbloses Öl

| Analyse: | | | |
|---|---|---|---|
| ber. | C 49,34 | H 5,61 | N 11,14 |
| gef. | C 49,44 | H 5,75 | N 11,06 |

### b) N,N,N',N'-Tetrakis-{2-[[N''-(carboxymethyl)]-N''-((N"'-methyl)-carbamoylmethyl)-amino]-ethyl}-meso-2,6-diaminopimelinsäure

In eine Lösung von 7,43 g (9,84 mmol) der nach Beispiel 8a) bereiteten Verbindung in wasserfreiem Tetrahydrofuran wird bei 0°C gasförmiges Methylamin bis zur Sättigung der Lösung eingeleitet. Anschließend läßt man den Ansatz 2 Stunden bei Raumtemperatur weiterrühren dampft ihn anschließend im Vakuum ein und trocknet den Rückstand am Ölpumpenvakuum.
Ausbeute: 8,65 g (100 % d. Th.) gelbliches Öl

| Analyse: | | | |
|---|---|---|---|
| ber. | C 47,83 | H 7,11 | N 15,94 |
| gef. | C 47,97 | H 7,24 | N 16,10 |

### c) Didysprosiumkomplex der N,N,N',N'-Tetrakis-{2-[[N''-(carboxymethyl)]-N'((N"'-methyl)-carbamoylmethyl)-amino]-ethyl}-meso-2,6-diaminopimelinsäure

8,51 g (9,69 mmol) des nach Beispiel 8b) hergestellten Tetraamids werden in 30 ml Wasser gelöst und mit 3,61 g (9,69 mmol) Dysprosiumoxid versetzt. Die Suspension wird solange auf 80 °C erhitzt bis eine Lösung vorliegt, die mit wenig sauren und basischen Ionenaustauschern vollständig entsalzt, dann filtriert und gefriergetrocknet wird.
Ausbeute: 11,24 g (96,9 % d. Th.) farbloses Lyophilisat

| Analyse (bezogen auf wasserfreie Substanz): | | | | |
|---|---|---|---|---|
| ber. | C 35,09 | H 4,71 | N 11,69 | Dy 27,13 |
| gef. | C 35,18 | H 4,81 | N 11,78 | Dy 27,04 |

### Beispiel 9

### Digadoliniumkomplex des Tetranatriumsalzes der N,N,N',N'-Tetrakis-{2-[N",N"-bis-(carboxymethyl)-amino]-2-(hydroxymethyl)-propyl}-meso-2,3-diaminobernsteinsäure

### a) 2,4-Dimethyl-4-brommethyl-2-oxazolin

Eine Lösung von 40,1 g (310 mmol) 2,4-Dimethyl-4-hydroxymethyl-2-oxazolin (J. Nys und J. Libeer, Bull. Soc. Chim. Belg., 65, 377 (1956)) und 85,5 g (326 mmol) Triphenylphosphin in 400 ml Dichlormethan wird bei 0°C portionsweise mit 58,0 g (326 mmol) N-Bromsuccinimid versetzt und anschließend über Nacht bei Raumtemperatur gerührt. Die Lösung wird eingedampft und der Rückstand mit tert.-Butyl-methylether verrieben. Es entsteht ein Niederschlag, der abgetrennt und mit tert.-Butyl-methylether gewaschen wird. Die vereinigten Filtrate werden bei Normaldruck einer fraktionierten Destillation unterzogen, wobei zunächst das Lösungsmittel abgezogen wird und anschließend bei einer Ölbadtemperatur von 200°C die Titelverbindung destilliert wird.
Ausbeute: 53,2 g (89,3 % d. Th.) farbloses Öl

| Analyse: | | | | |
|---|---|---|---|---|
| ber. | C 37,52 | H 5,25 | Br 41,60 | N 7,29 |
| gef. | C 37,49 | H 5,31 | Br 41,50 | N 7,34 |

### b) N,N,N',N'-Tetrakis-[(2,4-dimethyl-4,5-dihydrooxazol-4-yl)-methyl]-meso-2,3-diaminobernsteinsäure

11,6 g (78,3 mmol) meso-2,3-Diaminobernsteinsäure und 72,2 g (375 mmol) des nach Beispiel 9a) hergestellten Bromids werden in eine Suspension aus 80 ml Tetrahydroturan und 40 ml 2 n Phosphatpufferlösung (pH 8,0) eingetragen und bei Raumtemperatur 24 Stunden kräftig gerührt, wobei der pH-Wert der wäßrigen Phosphatpufferphase nach 2 und 8 Stunden auf den Ausgangswert eingestellt wird. Dann wird der pH-Wert der wässrigen Phase mit halbkonzentrierter Salzsäure auf pH 2 abgesenkt, die organische Phase abgetrennt und im Vakuum eingedampft. Der ölige Rückstand wird am Ölpumpenvakuum bei 50°C getrocknet und ohne weitere Reinigung weiter umgesetzt.
Ausbeute: 47,8 g Rohprodukt.

### c) N,N,N',N'-Tetrakis-[(2-amino-3-hydroxy-2-methyl)-propyl]-meso-2,3-diaminobernsteinsäure-Hexahydrochlorid

47,8 g des nach Beispiel 9b) hergestellten rohen Tetraoxazolinderivats werden in 150 ml Methanol aufgenommen und mit 50 ml konzentrierter Salzsäure versetzt. Man kocht das Reaktionsgemisch 3 Stunden unter Rückfluß und dampft anschließend bis zur Trockne ein. Der Rückstand wird ohne Reinigung weiter umgesetzt.
Ausbeute: 57,4 g Rohprodukt

### d) N,N,N',N'-Tetrakis-[(2-amino-3-hydroxy-2-methyl)-propyl]-meso-2,3-diaminobernsteinsäuredibenzylester-hexa-p-toluolsulfonat

57,4 g des nach Beispiel 9c) hergestellten rohen Hexahydrochlorids, 65,6 g (345 mmol) p-Toluolsulfonsäure und 84,7 g (783 mmol) Benzylalkohol werden in 300 ml 1,2-Dichlorethan am Wasserabscheider 1 d unter Rückfluß gekocht. Der gebildete Niederschlag wird abgetrennt, mit Diethylether gewaschen und im Vakuum getrocknet. Das Rohprodukt wird ohne Reinigung weiter umgesetzt.
Ausbeute: 135,4 g

### e) N,N,N',N'-Tetrakis-{2-[N'',N''-bis((benzyloxycarbonyl)-methyl)-amino]-2-(hydroxymethyl)-propyl}-meso-2,3-diaminobernsteinsäure-dibenzylester

60,0 g (ca 35 mmol) des nach Beispiel 9d) hergestellten rohen p-Toluolsulfonats und 79,0 g (345 mmol) Bromessigsäurebenzylester werden in 100 ml Tetrahydrofuran vorgelegt und mit 71,7 (517 mmol) Triethylamin versetzt. Der Ansatz wird bei Raumtemperatur 24 Stunden bei 60°C gerührt, anschließend eingedampft, in Essigsäureethylester aufgenommen und gegen konzentrierte, wäßrige Natriumhydrogencarbonatlösung ausgeschüttelt. Die organische Phase wird über Magnesiumsulfat getrocknet, im Vakuum eingedampft und der Rückstand an Kieselgel mit Hexan/Essigsäureethylester/Triethylamin (3:1:0,01) chromatographiert. Die produkthaltigen Fraktionen werden im Vakuum eingedampft.
Ausbeute: 27,0 g (42,1 % d. Th. über 4 Stufen) farbloses Öl.

| Analyse (bezogen auf lösungsmittelfreie Substanz): | | | |
|---|---|---|---|
| ber. | C 68,37 | H 6,50 | N 4,51 |
| gef. | C 68,41 | H 6,55 | N 4,48 |

### f) N,N,N',N'-Tetrakis-{2-[N",N"-bis(carboxymethyl)-amino]-2-(hydroxymethyl)-propyl}-meso-2,3-diaminobernsteinsäure

25,8 g (13,8 mmol) des Benzylethers aus Beispiel 9e) werden in 150 ml Methanol gelöst und nach Zugabe von 2,6 g Palladium (10 %) auf Aktivkohle unter Wasserstoffatmosphäre bei Raumtemperatur bis zur beendeten Wasserstoffaufnahme hydriert. Nach Filtrieren und Eindampfen des Filtrats im Vakuum erhält man ein farbloses Öl.
Ausbeute: 13,3g (100,0 % d. Th.)

| Analyse: | | | |
|---|---|---|---|
| ber. | C 45,00 | H 6,50 | N 8,75 |
| gef. | C 45,12 | H 6,61 | N 8,69 |

### g) Digadoliniumkomplex des Tetranatriumsalzes der N,N,N',N'-Tetrakis-{2-[N",N"-bis-(carboxymethyl)-amino]-2(hydroxymethyl)-propyl}-meso-2,3-diaminobernsteinsäure

10,5 g (10,9 mmol) der in Beispiel 9f) beschriebenen Decasäure werden in 100 ml Wasser aufgenommen, mit 3,96 g (12,9 mmol) Gadoliniumoxid versetzt und 3 Stunden bei 60°C gerührt. Anschließend stellt man mit verdünnter Natronlauge pH 7,2 ein, filtriert und gefriertrocknet das Filtrat.
Ausbeute: 14,4 g (97,4 % d. Th.) farbloses Lyophilisat

| Analyse (bezogen auf wasserfreie Substanz): | | | | | |
|---|---|---|---|---|---|
| ber. | C 31,86 | H 3,71 | Gd 23,17 | N 6,19 | Na 6,78 |
| gef. | C 31,95 | H 3,84 | Gd 23,05 | N 6,22 | Na 6,63 |

### Beispiel 10

### Digadoliniumkomplex des Dinatriumsalz der 3,6,11,14-Tetraaza-3,14-bis(carboxymethyl)-6,11-bis-[2-(N,N-bis-(carboxymethyl)-amino)-ethyl]-8,9-dihydroxy-hexadecan-1,16-disäure

### a) 4,5-Bis-{N,N,N',N'-tetrakis-[2-[(N",N"-bis(-tert.-butyloxycarbonylmethyl))-amino]-ethyl]-aminomethyl}-2,2-dimethyl-1,3-dioxolan

8,05 g (50,2 mmol) 4,5-Bis-(aminomethyl)-2,2-dimethyl-1,3-dioxolan und 79,3 g (225 mmol) N,N-Bis-[tert.-butyloxycarbonylmethyl]-2-bromethylamin (M. Williams und H. Rapoport, J. Org. Chem. 58, 1151 (1993)) werden in 90 ml Acetonitril vorgelegt und mit 70 ml 2 n Phosphatpufferlösung (pH 8,0) versetzt. Der Ansatz wird bei Raum-temperatur 30 Stunden kräftig gerührt, wobei die wäßrige Phosphatpufferphase nach 2,8 und 24 Stunden gegen frische Pufferlösung ausgetauscht wird. Dann wird die organische Phase im Vakuum eingedampft und der Rückstand an Kieselgel mit Hexan/Essigsäureethylester/Triethylamin (3:1:0,01) chromatographiert. Die produkthaltigen Fraktionen werden im Vakuum eingedampft.
Ausbeute: 36,9 g (59 % d. Th.) farbloser Feststoff.

| Analyse (unter Berücksichtigung des Lösemittelgehaltes): | | | |
|---|---|---|---|
| ber. | C 60,75 | H 9,39 | N 6,75 |
| gef. | C 60,53 | H 9,55 | N 6,49 |

### b) 3,6,11,14-Tetraaza-3,14-bis-(carboxymethyl)-6,11-bis-[2-((N,N-bis-carboxymethyl)-amino)-ethyl]-8,9-dihydroxy-hexadecan-1,16-disäure

12,8 g (10,3 mmol) des Octaesters aus Beispiel 10a) werden in 80 ml Methanol gelöst und bei Raumtemperatur mit 5,27 g (132 mmol) Natriumhydroxid in 8 ml Wasser versetzt. Anschließend wird die Reaktionsmischung auf 60°C erwärmt und 5 Tage gerührt. Nach vollständiger Verseifung wird das Lösungsmittel abdestilliert und der Rückstand in 150 ml Wasser aufgenommen. Der pH-Wert wird mit saurem Ionenaustauscher auf 1 eingestellt und der Ansatz 2 Stunden bei 50°C bis zur vollständigen Umsetzung gerührt. Nach dem Filtrieren wird die klare, wäßrige Lösung eingedampft und das erhaltene Produkt mit Isopropanol ausgerührt. Nach Filtrieren und Eindampfen des Filtrats im Vakuum erhält man ein farbloses Öl.
Ausbeute: 6,8 g (90 % d. Th.).

| Analyse (unter Berücksichtigung des Lösemittelgehaltes): | | | |
|---|---|---|---|
| ber. | C 44,44 | H 6,39 | N 11,11 |
| gef. | C 44,21 | H 6,50 | N 10,98 |

### c) Digadoliniumkomplex des Dinatriumsalzes der 3,6,11,14-Tetraaza-3,14-bis-(carboxymethyl)-6,11-bis-[2-(N,N-bis(carboxymethyl)-amino)-ethyl]-8,9-dihydroxy-hexadecan-1,16-disäure

5,9 g (7,8 mmol) der Decasäure aus Beispiel 10b) werden in 100 ml Wasser aufgenommen, mit 1,41 g (3,9 mmol) Gadoliniumoxid versetzt und 5 Stunden bei 55°C gerührt. Anschließend stellt man mit verdünnter Natronlauge pH 7,2 ein, filtriert und gefriertrocknet das Filtrat.
Ausbeute: 8,2 g (94,8 % d. Th.) farbloses Lyophilisat.

| Analyse (bezogen auf lösungsmittelfreie Substanz): | | | | | |
|---|---|---|---|---|---|
| ber. | C 30,32 | H 3,64 | Gd 28,35 | N 7,58 | Na 4,15 |
| gef. | C 30,21 | H 3,88 | Gd 28,09 | N 7,43 | Na 4,04 |

### Beispiel 11

### a) 1,4-Bis-[1-ethoxycarbonyl-2-(2-phthalimidoethyl)-4(N-phthaloyl)-amino-2-aza-butyl]-benzol

109,52 g (301,4 mmol) Bis(2-phthalimidoethyl)-amin, hergestellt nach E.V. Gramin, J. Org. Chem. USSR 23 (1987): 330, werden mit 41,0 g (100,5 mmol) 1,4-Phenylenbis-(α -bromessigsäureethylester), 41,66 g (301,4 mmol) Kaliumcarbonat und einer Spatelspitze Natriumiodid in 600 ml Dimethylformamid 5 Stunden auf 100 °C erhitzt.

Man dampft im Vakuum ein, nimmt mit 800 ml Wasser auf und extrahiert zweimal mit 500 ml Essigester. Die organische Phase wird über Magnesiumsulfat getrocknet und im Vakuum eingedampft. Der Rückstand wird an Kieselgel chromatographiert [Laufmittel: Hexan/Aceton (3:1)].
Ausbeute: 36,17 g (37 % der Theorie bzgl. des Esters).

| Analyse: | | | |
|---|---|---|---|
| ber. | C 66,66 | H 4,97 | N 8,64 |
| gef. | C 66,59 | H 5,04 | N 8,58 |

### b) 1,4-Bis-[3-oxo-1-(2-aminoethyl)-piperazin-2-yl]-benzol

36,0 g (37 mmol) der Titelverbindung aus Beispiel 11a) werden in 350 ml Methanol mit 22,2 ml (370 mmol) 80 %iger Hydrazinhydrat-Lösung versetzt und 8 Stunden am Rückfluß erhitzt. Man kühlt auf 0 °C im Eisbad und saugt vom ausgefallenen Niederschlag ab. Das Filtrat wird zur Trockne eingedampft und der Rückstand an Kieselgel chromatographiert [Laufmittel: Methylenchlorid/Methanol/33 %ige Ammoniaklösung aq. (4:2:1)].
Ausbeute: 10,94 g (82 % der Theorie) eines hellgelben Öls.

| Analyse: | | | |
|---|---|---|---|
| ber. | C 59,98 | H 7,83 | N 23,31 |
| gef. | C 59,93 | H 7,75 | N 23,25 |

### c) 1,4-Bis-[3-oxo4-tert.-butoxycarbonylmethyl-1-(2-N,N-bis(tert.-butoxy-carbonylmethyl)-aminoethyl)-piperazin-2-yl]-benzol

10,5 g (29,13 mmol) der Titelverbindung aus Beispiel 11b) werden in 400 ml Tetrahydrofuran unter einer Argonatmosphäre gelöst. Man gibt 21,84 g (728,2 mmol) 80 %iges Natriumhydrid und 142,1 g (728,2 mmol) Bromessigsäure-tert.-butylester hinzu und erwärmt 2 Tage bei 60 °C. Man kühlt im Eisbad und setzt vorsichtig Wasser zu. Anschließend wird im Vakuum zur Trockne eingedampft und der Rückstand in 400 ml Wasser aufgenommen. Man extrahiert dreimal mit 150 ml Methylenchlorid, trocknet die organische Phase über Methylenchlorid und engt im Vakuum ein. Der Rückstand wird an Kieselgel chromatographiert [Laufmittel: Methylenchlorid/Methanol (20:1)].
Ausbeute: 19,49 g (64 % der Theorie) eines farblosen Öls.

| Analyse: | | | |
|---|---|---|---|
| ber. | C 62,05 | H 8,48 | N 8,04 |
| gef. | C 61,97 | H 8,53 | N 8,00 |

### d) 1,4-Bis-[3-oxo-carboxymethyl-1-(2-N,N-bis(carboxymethyl)aminoethyl)-piperazin-2-yl]-benzol

19,5 g (18,65 mmol) der Titelverbindung aus Beispiel 11c) werden in 150 ml Trifluoressigsäure gelöst und 1 Stunde bei Raumtemperatur gerührt. Man dampft im Vakuum zur Trockne ein und kristallisiert den Rückstand aus Methanol/Aceton um.
Ausbeute: 11,77 g (89 % der Theorie) eines cremefarbenen Pulvers.

| Analyse: | | | |
|---|---|---|---|
| ber. | C 50,84 | H 5,69 | N 11,86 |
| gef. | C 50,78 | H 5,73 | N 11,81 |

### e) 1,4-Bis-[1-carboxy-2(2-N,N-bis(carboxymethyl)aminoethyl)-5,5-bis(carboxymethyl)-2,5-diazapentyl]-benzol

11,5 g (16,23 mmol) der Titelverbindung aus Beispiel 11d) werden in 300 ml 5N Natronlauge gelöst und 12 Stunden unter Rückfluß erhitzt. Man läßt auf 50 °C abkühlen und versetzt mit 244 ml 1N Bromessigsäure in Tetrahydrofuran. Dann wird 24 Stunden bei 50 °C gerührt. Die Lösung wird zur Trockne eingedampft und anschließend an Kieselgel chromatographiert [Laufmittel: Ethanol/konz. aq. Ammoniak/Wasser (4:1:1)]. Nach Eindampfen der Hauptfraktionen wird das Ammoniumsalz über eine Säule mit saurem Ionenaustauscher gegeben und das Eluat gefrier-getrocknet.
Ausbeute: 8,66 g (62 % der Theorie) eines farblosen amorphen Pulvers.

| Analyse (auf Wasser korrigiert): | | | |
|---|---|---|---|
| ber. | C 47,44 | H 5,62 | N 9,76 |
| gef. | C 47,40 | H 5,69 | N 9,71 |

### f) Digadoliniumkomplex von 1,4-Bis-[1-carboxy-2-(2-N,N-bis(carboxymethyl)-aminoethyl)-5,5-bis(carboxymethyl)-2,5-diazapentyl]-benzol [als Tetranatriumsalz]

8,5 g (9,87 mmol) der Titelverbindung aus Beispiel 11e) und 3,58 g (9,87 mmol) Gadoliniumoxid werden in 80 ml Wasser 30 Minuten bei 80 °C gerührt. Man kühlt auf Raumtemperatur ab und stellt durch Zugabe von 2N Natronlauge auf pH 7,2 ein. Es wird 30 Minuten mit etwas Aktivkohle bei Raumtemperatur gerührt und anschließend filtriert. Das Filtrat wird gefriergetrocknet.
Ausbeute: 12,41 g (100 % der Theorie) eines farblosen amorphen Pulvers.

| Analyse (auf Wasser korrigiert): | | | | | |
|---|---|---|---|---|---|
| ber. | C 32,48 | H 3,05 | N 6,68 | Gd 25,02 | Na 7,31 |
| gef. | C 32,43 | H 3,11 | N 6,63 | Gd 24,96 | Na 7,36 |

### g) Diytterbium-Komplex von 1,4-Bis-[1-carboxy-2-(2-N,N-bis(carboxymethyl)-aminoethyl)-5,5-bis(carboxymethyl)-2,5-diazapentyl]-benzol [als Tetranatriumsalz]

8,5 g (9,87 mmol) der Titelverbindung aus Beispiel 11e) und 3,89 g (9,87 mmol) Ytterbiumoxid werden in 80 ml Wasser 4 Tage bei 80 °C gerührt. Man kühlt auf Raumtemperatur ab und stellt durch Zugabe von 2N Natronlauge auf pH 7,2 ein. Es wird 30 Minuten mit etwas Aktivkohle bei Raumtemperatur gerührt und anschließend filtriert. Das Filtrat wird gefriergetrocknet.
Ausbeute: 12,72 g (100 % der Theorie) eines farblosen amorphen Pulvers.

| Analyse (auf Wasser korrigiert): | | | | | |
|---|---|---|---|---|---|
| ber. | C 31,69 | H 2,97 | N 6,52 | Yb 26,85 | Na 7,14 |
| gef. | C 31,43 | H 3,10 | N 6,48 | Yb 26,71 | Na 6,98 |

### h) Dimangan(II)-Komplex von 1,4-Bis-[1-carboxy-2-(2-N,N-bis(carboxymethyl)-aminoethyl)-5,5-bis(carboxymethyl)-2,5-diazapentyl]-benzol [als Hexanatriumsalz]

8,5 g (9,87 mmol) der Titelverbindung aus Beispiel 11e) und 1,13 g (9,87 mmol) Mangan(II)carbonat werden in 80 ml Wasser 2 Stunden bei 80 °C gerührt. Man kühlt auf Raumtemperatur ab und stellt durch Zugabe von 2N Natronlauge auf pH 7,2 ein. Es wird 30 Minuten mit etwas Aktivkohle bei Raumtemperatur gerührt und anschließend filtriert. Das Filtrat wird gefriergetrocknet.
Ausbeute: 10,84 g (100 % der Theorie) eines farblosen amorphen Pulvers.

| Analyse (auf Wasser korrigiert): | | | | | |
|---|---|---|---|---|---|
| ber. | C 37,18 | H 3,49 | N 7,65 | Mn 10,00 | Na 12,56 |
| gef. | C 37,03 | H 3,58 | N 7,55 | Mn 9,89 | Na 12,40 |

### Beispiel 12

### Herstellung eines Kontrastmittels für die nuclearmedizinische Anwendung:

### Indium-111-Komplex der N,N'-bis-{2-[N'',N"-bis-(carboxymethyl)-amino]-3-[(4-methoxy)-phenyl]-propyl}-N,N"-bis-{2-[N"',N"'-bis-(carboxymethyl)-amino]-ethyl}-meso-2,3-diaminobernsteinsäure

1,0 ml einer salzsauren Lösung von 1,0 mCi Indium-111-chlorid werden mit gesättigter, wässriger Natrimhydrogencarbonatlösung auf pH 7,5 eingestellt. Man gibt 2,0 mg (2,0 µmol) der nach Beispiel 6g) hergestellten Decasäure zu, filtriert die Probe über ein Membranfilter und autoklaviert das Filtrat in einer geschlossenen Glasampulle. Die Lösung ist gebrauchsfertig.

### Analytik:

Durch HPLC der fertigen Probe über Kieselgel-RP-18 mit Phosphatpufferlösung kann mittels eines Gammastrahlendetektors gezeigt werden, daß praktisch 100 % der eingesetzten Aktivität im Komplex enthalten sind.

### Beispiel 13

### Dihafniumkomplex des N,N,N',N'-Tetrakis-{2-[N",N"-bis(carboxymethyl)-amino]-ethyl}-2,7-diaminooctandisäure-N"',N""-bis(2-methoxyethyl)-diamid

### a) 2,7-Di(benzyloxycarbonylamino)octandisäuredimethylester

9,45 g (20 mmol) 2,7-Di(benzyloxycarbonylamino)octandisäure (P.M. Fischer et al. TH 50,2277 (1994)) werden in 100 ml Methanol gelöst und mit 14,6 ml (200 mmol) Thionylchlorid bei 0°C versetzt und anschließend über Nacht bei Raumtemperatur gerührt. Man dampft zur Trockne ein und nimmt den Rückstand in Essigsäureethylester auf. Bei 0°C wird mit gesättigter Natriumhydrogencarbonatlösung versetzt und die organische Phase abgetrennt. Die wäßrige Phase wird dreimal mit je 50 ml Essigsäureethylester extrahiert, die vereinigten organischen Phasen werden über Kaliumcarbonat getrocknet, filtriert und eingeengt.
Ausbeute: 9,2 g (91,9 % d. Th.)

| Analyse (bezogen auf lösungsmittelfreie Substanz): | | | | |
|---|---|---|---|---|
| ber. | C 62,39 | H 6,44 | N 5,60 | O 25,57 |
| gef. | C 62,21 | H 6,60 | N 5,73 | |

### b) 2,7-Di(benzyloxycarbonylamino)octandisäure-bis(2-methoxyethyl)-diamid

Eine Lösung von 9,05 g (18,1 mmol) 2,7-Di(benzyloxycarbonylamino)octandisäure-dimethylester in 30 ml Ethanol wird mit 6,79 g (90,4 mmol) 2-Methoxyethylamin versetzt und 14 Stunden unter Rückfluß gekocht. Anschließend wird vollständig eingedampft und der Rückstand an Kieselgel mit Dichlormethan/Methanol chromatographiert. Nach dem Eindampfen der produkthaltigen Fraktionen erhält man ein leicht gelbliches Öl.
Ausbeute: 9,1 g (86,7 % d. Th.)

| Analyse (bezogen auf lösungsmittelfreie Substanz): | | | | |
|---|---|---|---|---|
| ber. | C 61,42 | H 7,22 | N 9,55 | O 21,82 |
| gef. | C 61,28 | H 7,42 | N 9,62 | |

### c) 2,7-Diaminooctandisäure-bis-(2-methoxyethyl)-diamid

8,9 g (15 mmol) Diamid aus Beispiel 13b) werden in 30 ml Methanol gelöst und zwei Stunden bei Normaldruck über 0,9 g Pd/C (10%) bei Raumtemperatur hydriert. Nach beendeter Reaktion wird der Katalysator abfiltriert und der Rückstand im Vakuum zur Trockne eingedampft.
Ausbeute: 4,3 g (90 % d. Th.) gelbliches Öl.

| Analyse (bezogen auf lösungsmittelfreie Substanz): | | | | |
|---|---|---|---|---|
| ber. | C 52,81 | H 9,50 | N 17,59 | O 20,10 |
| gef. | C 52,68 | H 9,62 | N 17,33 | |

### d) N,N,N',N'-Tetrakis-{2-[N",N"-bis-(benzyloxycarbonylmethyl)-amino]-ethyl}-2,7-diaminooctandisäure-N"',N""-bis-(2-methoxyethyl)-diamid

4,2 g (13,2 mmol) der nach Beispiel 13c) hergestellten Verbindung und 24,9 g (59,4 mmol) N,N-Bis-[(benzyloxycarbonyl)-methyl]-2-bromethylamin (M. Williams und H. Rapoport, J. Org. Chem. 58, 1151 (1993)) werden in 30 ml Acetonitril vorgelegt und mit 22 ml 2 n Phosphatpufferlösung (pH 8,0) versetzt. Der Ansatz wird bei Raumtemperatur 20 Stunden kräftig gerührt, wobei die wäßrige Phosphatpufferphase nach 2 und 8 Stunden gegen frische Pufferlösung ausgetauscht wird. Dann wird die organische Phase im Vakuum eingedampft und der Rückstand an Kieselgel mit Hexan/Essigsäureethylester/Triethylamin (3:1:0,01) chromatographiert. Die produkthaltigen Fraktionen werden im Vakuum eingedampft.
Ausbeute: 17,6 g (79,5 % d. Th.) farbloses Öl.

| Analyse (bezogen auf lösungsmittelfreie Substanz): | | | | |
|---|---|---|---|---|
| ber. | C 67,37 | H 6,86 | N 6,69 | O 19,09 |
| gef. | C 67,19 | H 6,92 | N 6,74 | |

### e) Dihafniumkomplex des N,N,N',N'-Tetrakis-{2-[N",N"-bis(carboxymethyl)-amino]-ethyl}-2,7-diaminooctandisäure-N"',N""-bis(2-methoxyethyl)-diamid

17,4 g (10,4 mmol) des in Beispiel 13d) beschriebenen Octaesters werden in 80 ml Methanol gelöst und mit 62 ml 2 n Natronlauge versetzt. Man kocht 1,5 Stunden unter Rückfluß, zieht das Methanol im Vakuum ab und rührt weitere 3 Stunden bei 60°C. Anschließend stellt man mit konzentrierter Salzsäure pH 1 ein, dampft im Vakuum bis zur Trockne ein und rührt den Rückstand mit Isopropanol aus. Nach Filtrieren und Eindampfen des Filtrats im Vakuum erhält man ein farbloses Öl, welches in 80 ml Wasser aufgenommen wird und mit 5,1 g (20,8 mmol) Hafniumhydroxid (hergestellt aus Hafniumoxychloridoctahydrat nach der Vorschrift von D. J. Williams et al., J. Chem. Soc. Dalton Trans. 2475, 1992) versetzt wird. Man kocht 72 Stunden am Rückfluß, rührt anschließend die wäßrige Lösung mit 3,5 g Aktivkohle und filtriert. Das Filtrat wird etwas eingeengt und gefriergetrocknet.
Ausbeute: 12,3 g (90,7.% d Th.) farbloses Lyophilisat.

| Analyse (bezogen auf lösungsmittelfreie Substanz): | | | | | |
|---|---|---|---|---|---|
| ber. | C 35,00 | H 4,48 | N 8,59 | O 24,54 | Hf 27,38 |
| gef. | C 34,86 | H 4,69 | N 8,37 | | Hf 27,11 |

### Beispiel 14

### Diytterbiumkomplex des Dinatriumsalzes der N,N'-bis- {2-[N",N"-bis(carboxymethyl)-amino]-3-[(4-ethoxy)-phenyl]-propyl}-N,N'-bis-{2-[N"',N"'-bis-(carboxymethyl)-amino]-ethyl}-meso-2,3-diaminobernsteinsäure-N"",N""-bis(2-methoxyethyl)-diamid

meso-2,3-Diaminobernsteinsäure-N,N'-bis(2-methoxyethyl)-diamid, Diacetat (Verbindung nach Beispiel 4a) wird analog den in Beispiel 6a beschriebenen Bedingungen in meso-2,3-Di-(benzylamino)-bernsteinsäure-N',N'-bis(2-methoxyethyl)-diamid überführt und analog den Beispiel 6b beschriebenen Bedingungen mit N,N-Bis-[(tert.-butyloxycarbonyl)-methyl]-2-bromethylamin dialkyliert. Das Alkylierungsprodukt wird nun entsprechend den in Beispiel 6c beschriebenen Bedingungen hydrogenolytisch debenzyliert und anschließend mit analog Beispiel 6d - 6e bereitetem 1-Brom-2-[N,N-bis((tert.-butyloxycarbonyl)-methyl)-amino]-3-[(4-ethoxy)-phenyl]-propan entsprechend den in Beispiel 6f angegebenen Bedingungen dialkyliert. Der so erhaltenene Octaester wird nun analog den in Beispiel 6g und 6h beschriebenen Bedingungen hydrolysiert und unter Verwendung von Ytterbiumcarbonat in die Titelverbindung überführt.

| Analyse (bezogen auf wasserfreie Substanz): | | | | | | |
|---|---|---|---|---|---|---|
| ber. | C 40,26 | H 4,55 | N 7,22 | O 22,69 | Na 2,96 | Yb 22,31 |
| gef. | C 40,30 | H 4,53 | N 7,09 | O 22,77 | Na 3,12 | Yb 22,15 |

## Patentansprüche

1. Verbindungen der allgemeinen Formel I, bestehend aus den Inkrementen (A) und (B): 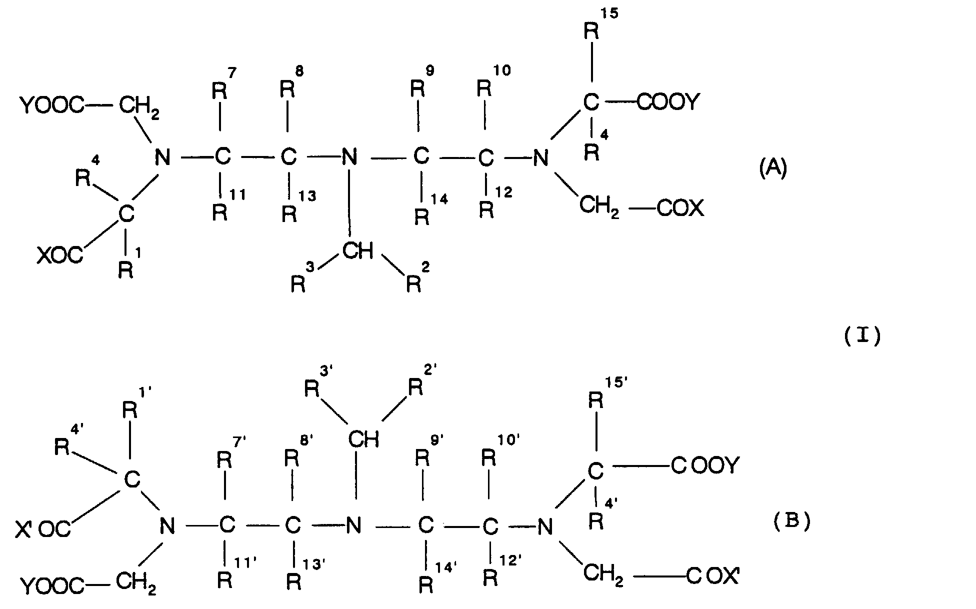
wobei die Inkremente A und B über eines der Substituentenpaare R¹ und R^{1'}, R² und R^{2'}, R¹⁵ und R^{15'}, R¹ und R^{2'}, R^{1'} und R², R^{1'} und R¹⁵, R¹ und R^{15'}, R² und R^{15'}, R^{2'} und R¹⁵ miteinander verknüpft sind,
worin das jeweils verknüpfende Substituentenpaar für Heterocyclus, einen Phenylenrest, eine C₀ - C₃₀-Alkylen- oder eine C₇- C₃₀ Aralkylenkette stehen, die gegebenenfalls durch 1 - 4 Hydroxy-, C₁ - C₆-Alkoxy-, Carboxy- oder Mercaptogruppen substituiert und/oder durch 1 bis 6 Sauerstoff-, Stickstoff-, Schwefelatome, Sulfinyl- und/oder Sulfonylgruppen unterbrochen ist,
und die für die Verknüpfung nicht benötigten Substituenten R¹, R^{1'}, R¹⁵ und R^{15'} unabhängig voneinander für ein Wasserstoffatom oder eine gegebenenfalls durch 1 - 4 Hydroxy- oder Mercaptogruppen substituierte C₁ - C₆-Alkylgmppe stehen, und die für die Verknüpfung nicht benötigten Substituenten R² und R^{2'} unabhängig voneinander für ein Wasserstoffatom, eine C₁ - C₆-Alkylgruppe, eine Gruppe -(CH₂)ₙOH oder -(CH₂)ₙSH mit n = 1 oder 2 stehen,
und die Substituenten R³ und R^{3'} unabhängig voneinander für eine Gruppe COOY-oder für eine CONR⁵R⁶-Gruppe stehen, worin R⁵ und R⁶ unabhängig voneinander eine C₁ - C₆-Alkylgruppe, die gegebenenfalls durch 1 - 4 Hydroxy- oder C₁ - C₆-Alkoxygruppen substituiert und/oder durch 1 - 6 Sauerstoff-, Stickstoff- und/oder Schwefelatome unterbrochen ist, bedeuten oder gemeinsam unter Einbeziehung des Stickstoffatoms einen 5- oder 6-Ring bilden, der gegebenenfalls ein Sauerstoffatom, ein weiteres acyliertes Stickstoffatom oder eine Sulfonylgruppe enthält und/oder mit 1 - 3 Hydroxygruppen substituiert ist,
und die Substituenten R⁴ und R^{4'} für ein Wasserstoffatom und/oder eine C₁ - C₆-Alkylgruppe stehen,
und die Substituenten R⁷ - R¹⁴ und R^{7'} - R^{14'} für ein Wasserstoffatom, eine Phenylgruppe, eine C₀ - C₃₀-Alkylen- oder eine C₇- C₃₀ Aralkylenkette stehen, die gegebenenfalls durch 1 - 4 Hydroxy-, C₁ - C₆-Alkoxy- oder Mercaptogruppen substituiert und/oder durch 1 - 6 Sauerstoff-, Stickstoff- und/oder Schwefelatome unterbrochen ist,
oder die Substituenten R⁷ und R⁸, R^{7'} und R^{8'} oder R⁹ und R¹⁰ und R^{9'} und R^{10'} gemeinsam eine Tri- oder Tetramethylengruppe bilden,
und Y jeweils für ein Wasserstoffatom und/oder ein Metallionenäquivalent eines Elements der Ordnungszahlen 21 - 32, 37 - 39, 42 - 51 und 57 - 83 steht,
und X und X' für eine Gruppe -OY, oder -CONR⁵R⁶ stehen, mit Y, R⁵ und R⁶ in der genannten Bedeutung,
sowie deren Salze mit anorganischen und/oder organischen Basen oder Aminosäuren.

2. Verbindungen gemäß Anspruch 1, dadurch gekennzeichnet, daß Y für ein Wasserstoffatom steht.

3. Verbindungen gemäß Anspruch 1, dadurch gekennzeichnet, daß mindestens zwei der Substituenten Y Metallionenäquivalente mindestens eines Elements der Ordnungszahlen 21 - 32, 37 - 39, 42 - 51 und 57 - 83 bedeuten.

4. Verbindungen gemäß Anspruch 1, dadurch gekennzeichnet, daß die Verknüpfung der Inkremente (A) und (B) jeweils über die Substituentenpaare R¹, R^{1'}; R², R^{2'}; R¹⁵, R^{15'}; R¹, R^{2'} oder R^{1'}, R² erfolgt.

5. Verbindungen gemäß Anspruch 1, dadurch gekennzeichnet, daß das Substituentenpaar R², R^{2'} eine direkte Bindung, eine C₁ - C₃₀-Alkylenkette, eine C₇ - C₃₀-Aralkylenkette, die durch zwei Sauerstoffatome unterbrochen ist, oder ein Phenylenrest darstellt.

6. Verbindungen gemäß Anspruch 1, dadurch gekennzeichnet, daß das Substituentenpaar R¹, R^{1'} eine durch ein Sauerstoffatom unterbrochene C₂ - C₃₀-Alkylenkette darstellt.

7. Verbindungen gemäß Anspruch 1, dadurch gekennzeichnet, daß das Substituentenpaar R¹, R^{2'} eine durch ein Sauerstoffatom unterbrochene C₇ - C₃₀-Aralkylenkene darstellt.

8. Diagnostische Mittel, enthaltend mindestens eine Komplexverbindung gemäß Anspruch 1, gegebenenfalls mit in der Galenik üblichen Zusätzen.

9. Verfahren zur Herstellung der Verbindungen gemäß Anspruch 1, in denen Y jeweils für ein Metallionenäquivalent eines Elements der Ordnungszahlen 21 - 32, 37 - 39, 42 - 51 und 57 - 83 steht, dadurch gekennzeichnet, daß man die Verbindungen der Formel I nach Anspruch 1, in denen Y für ein Wasserstoffatom steht, mit einem Metallsalz oder Metalloxid eines Elementes der genannten Ordnungszahlen umsetzt.

10. Verwendung von mindestens einem Metallkomplex nach Anspruch 1 für die Herstellung von Mitteln für die NMR- und/oder Röntgendiagnostik.

11. Verwendung von mindestens einem Metallkomplex nach Anspruch 1 für die Herstellung von Mitteln für die Radiodiagnostik und/oder Radiotherapie.

12. Metallkomplex nach Anspruch 1, nämlich der Digadoliniumkomplex des Tetranatriumsalzes der N,N,N',N'-Tetrakis-{2-[N",N"-bis-(carboxymethyl)-amino]-ethyl}-meso-2,3-diaminobernsteinsäure.

13. Metallkomplex nach Anspruch 1, nämlich der Digadoliniumkomplex des Tetranatriumsalzes der N,N,N',N'-Tetrakis-{2-[N",N"-bis-(carboxymethyl)-amino]-ethyl}-meso-2,6-diaminopimelinsäure.

14. Metallkomplex nach Anspruch 1, nämlich der Diytterbiumkomplex des Tetranatriumsalzes der 3-{4-[2-[N,N-bis-[2-[N',N'-bis(carboxymethyl)-amino]-ethyl]]-amino-2-carboxyethyl]-phenoxy}-2-{N-(2-[N'-[2-[N",N"-bis(carboxymethyl)-amino]-ethyl]-N'(carboxymethyl)-amino]-ethyl}-N-(carboxymethyl)-amino}-propionsäure.

15. Metallkomplex nach Anspruch 1, nämlich der Digadoliniumkomplex des Dinatriumsalzes des N,N,N',N'-Tetrakis-{2-[N",N"-bis(carboxymethyl)-amino]-ethyl}-meso-2,3-diaminobernsteinsäure-N"',N""-bis-(2-methoxyethyl)-diamids.

16. Metallkomplex nach Anspruch 1, nämlich der Digadoliniumkomplex des Tetranatriumnatriumsalzes des 1,8-Bis-{4-[2-(N,N-bis-(2-(N',N'-bis(carboxymethyl)-amino)-ethyl)-amino)-2-carboxyethyl]-phenoxy}-octans.

17. Metallkomplex nach Anspruch 1, nämlich der Didysprosiumkomplex des Tetranatriumsalzes der N,N'-bis-{2-[N",N"-bis-(carboxymethyl)-amino]-3-[(4-methoxy)-phenyl]-propyl}-N,N"-bis-{2-[N"',N"'-bis-(carboxymethyl)-amino]-ethyl}-meso-2,3-diaminobernsteinsäure.

18. Metallkomplex nach Anspruch 1, nämlich der Digadoliniumkomplex des Tetranatriumsalzes der 3,6,9,15,18,21-Hexaaza-10,14-bis-carboxy-3,6,9,15,18,21-hexakis-(carboxymethyl)-12-oxa-tricosan-1,23-disäure.

19. Metallkomplex nach Anspruch 1, nämlich der Didysprosiumkomplex der N,N,N',N'-Tetrakis-{2-[[N'-(carboxymethyl)]-N'-((N''-methyl)-carbamoylmethyl)-amino]-ethyl}-meso-2,6-diaminopimelinsäure.

20. Metallkomplex nach Anspruch 1, nämlich der Digadoliniumkomplex des Tetranatriumsalzes der N,N,N',N'-Tetrakis-{2-[N",N"-bis-(carboxymethyl)-amino]-2-(hydroxymethyl)-propyl}-meso-2,3-diaminobernsteinsäure.

21. Metallkomplex nach Anspruch 1, nämlich der Digadoliniumkomplex des Tetranatriumsalzes des 1,4-Bis-{1-carboxy-2-[2-N,N-bis(carboxymethyl)aminoethyl]-5,5-bis(carboxymethyl)-2,5-diazapentyl}-benzols.

22. Metallkomplex nach Anspruch 1, nämlich der Diytterbiumkomplex des Tetranatriumsalzes des 1,4-Bis-{1-carboxy-2-[2-N,N-bis(carboxymethyl)aminoethyl]-5,5-bis(carboxymethyl)-2,5-diazapentyl}-benzols.

23. Metallkomplex nach Anspruch 1, nämlich der Dimangankomplex des Hexanatriumsalzes des 1,4-Bis-{1-carboxy-2-[2-N,N-bis(carboxymethyl)aminoethyl]-5,5-bis(carboxymethyl)-2,5-diazapentyl}-benzols.

24. Metallkomplex nach Anspruch 1, nämlich der Indium-111-Komplex der N,N'-bis-{2-[N",N"-bis-(carboxymethyl)-amino]-3-[(4-methoxy)-phenyl]-propyl}-N,N'-bis-{2-[N"',N"'-bis-(carboxymethyl)-amino]-ethyl}-meso-2,3-diaminobernsteinsäure.

25. Metallkomplex nach Anspruch 1, nämlich der Dihafniumkomplex des N,N,N',N'-Tetrakis-{2-[N",N"-bis-(carboxymethyl)-amino]-ethyl}-2,7-diaminooctandisäure-N"",N""-bis(2-methoxyethyl)-diamids.

26. Metallkomplex nach Anspruch 1, nämlich der Diytterbiumkomplex des Dinatriumsalzes des N,N'-bis-{2-[N",N"-bis(carboxymethyl)-amino]-3-[(4-ethoxy)-phenyl}-N,N'-bis-{2-[N"',N"'-bis-(carboxymethyl)-amino]-ethyl}-meso-2,3-diaminobernsteinsäure-N"", N""'-bis-(2-methoxyethyl)-diamids.

## Claims

1. Compounds of the general formula I comprising the increments (A) and (B): 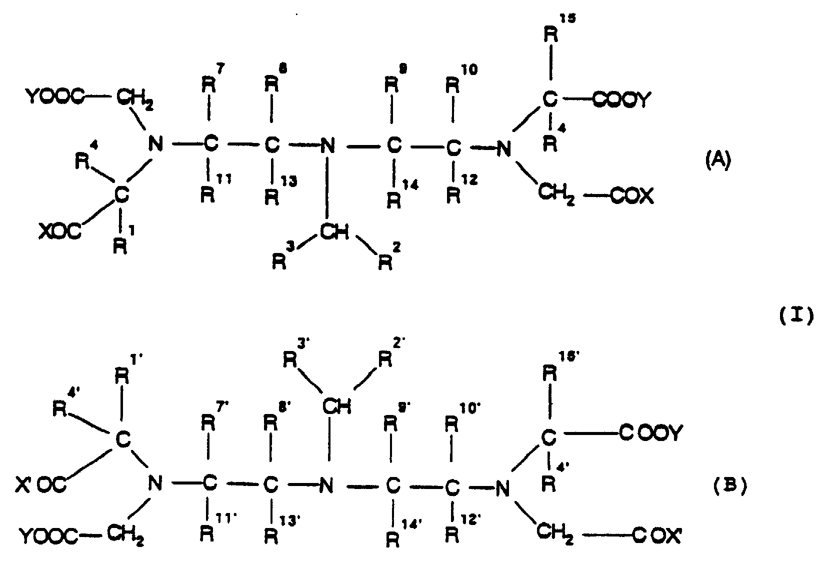
wherein the increments A and B are linked to one another via one of the substituent pairs R¹ and R^{1'}, R² and R^{2'}, R¹⁵ and R^{15'}, R¹ and R^{2'}, R^{1'} and R², R^{1'} and R¹⁵, R¹ and R^{15'}, R² and R^{15'}, R^{2'} and R¹⁵,
wherein the particular linking substituent pair represents a heterocyclic radical, a phenylene radical, a C₀-C₃₀-alkylene or a C₇-C₃₀-aralkylene chain, which is optionally substituted by 1-4 hydroxyl, C₁-C₆-alkoxy, carboxyl or mercapto groups and/or interrupted by 1 to 6 oxygen, nitrogen, sulphur atoms, sulphinyl and/or sulphonyl groups,
and the substituents R¹,R^{1'}, R¹⁵ and R^{15'} which are not required for the linking independently of one another represent a hydrogen atom or a C₁-C₆-alkyl group which is optionally substituted by 1-4 hydroxyl or mercapto groups,
and the substituents R² and R^{2'} which are not required for the linking independently of one another represent a hydrogen atom, a C₁-C₆-alkyl group, a group -(CH₂)ₙOH or -(CH₂)ₙSH, where n = 1 or 2,
and the substituents R³ and R^{3'} independently of one another represent a group COOY or represent a CONR⁵R⁶ group, wherein R⁵ and R⁶ independently of one another denote a C₁-C₆-alkyl group, which is optionally substituted by 1-4 hydroxyl or C₁-C₆-alkoxy groups and/or interrupted by 1-6 oxygen, nitrogen and/or sulphur atoms, or together, including the nitrogen atom, form a 5- or 6-membered ring, which optionally contains an oxygen atom, a further acylated nitrogen atom or a sulphonyl group and/or is substituted by 1-3 hydroxyl groups,
and the substituents R⁴ and R^{4'} represent a hydrogen atom and/or a C₁-C₆-alkyl group,
and the substituents R⁷-R¹⁴ and R^{7'}-R^{14'} represent a hydrogen atom, a phenyl group, a C₀-C₃₀-alkylene or a C₇-C₃₀-aralkylene chain, which is optionally substituted by 1-4 hydroxyl, C₁-C₆-alkoxy or mercapto groups and/or interrupted by 1-6 oxygen, nitrogen and/or sulphur atoms,
or the substituents R⁷ and R⁸, R^{7'} and R^{8'} or R⁹ and R¹⁰, and R^{9'} and R¹⁰ together form a tri- or tetramethylene group,
and Y in each case represents a hydrogen atom and/or a metal ion equivalent of an element of the atomic numbers 21-32,37-39,42-51 and 57-83,
and X and X represent a group -OY, or -CONR⁵R⁶, where Y, R⁵ and R⁶ have the meaning given,
and salts thereof with inorganic and/or organic bases or amino acids.

2. Compounds according to Claim 1, characterized in that Y represents a hydrogen atom.

3. Compounds according to Claim 1, characterized in that at least two of the substituents Y denote metal ion equivalents at least of one element of the atomic numbers 21 - 32, 37 - 39, 42 - 51 and 57 - 83.

4. Compounds according to Claim 1, characterized in that the linking of the increments (A) and (B) in each case takes place via the substituent pairs R¹, R^{1'}; R², R^{2'}; R¹⁵, R^{15'}; R¹, R^{2'} or R^{1'}, R².

5. Compounds according to Claim 1, characterized in that the substituent pair R², R^{2'} represents a direct bond, a C₁-C₃₀-alkylene chain, a C₇-C₃₀-aralkylene chain which is interrupted by two oxygen atoms or a phenylene radical.

6. Compounds according to Claim 1, characterized in that the substituent pair R¹, R^{1'} represents a C₂-C₃₀-alkylene chain interrupted by an oxygen atom.

7. Compounds according to Claim 1, characterized in that the substituent pair R¹, R^{2'} represents a C₇-C₃₀-aralkylene chain interrupted by an oxygen atom.

8. Diagnostic composition comprising at least one complex compound according to Claim 1, if appropriate with additives customary in pharmaceutical formulation.

9. Process for the preparation of the compounds according to Claim 1, in which Y in each case represents a metal ion equivalent of an element of the atomic numbers 21 - 32, 37 - 39, 42 - 51 and 57 - 83, characterized in that the compounds of the formula I according to Claim 1, in which Y represents a hydrogen atom, are reacted with a metal salt or metal oxide of an element of the atomic numbers mentioned.

10. Use of at least one metal complex according to Claim 1 for the preparation of compositions for NMR and/or X-ray diagnostics.

11. Use of at least one metal complex according to Claim 1 for the preparation of compositions for radio-diagnostics and/or radiotherapy.

12. Metal complex according to Claim 1, that is to say the digadolinium complex of the tetrasodium salt of N,N,N',N'-tetrakis-{2- [N",N"-bis-(carboxymethyl)-amino]-ethyl}-meso-2,3-diaminosuccinic acid.

13. Metal complex according to Claim 1, that is to say the digadolinium complex of the tetrasodium salt of N,N,N',N'-tetrakis-[2-[N",N"-bis-(carboxymethyl)-amino]-ethyl}-meso-2,6-diaminopimelic acid.

14. Metal complex according to Claim 1, that is to say the diytterbium complex of the tetrasodium salt of 3-{4-[2-[N,N-bis-[2-[N',N'-bis-(carboxymethyl)-amino]-ethyl]]-amino-2-carboxymethyl]-phenoxy}-2-{N-{2-[N'-[2-[N",N"-bis-(carboxymethyl)-amino]-ethyl'N'-(carboxymethyl)-amino]-ethyl}-N-(carboxymethyl)-amino}-propionic acid.

15. Metal complex according to Claim 1, that is to say the digadolinium complex of the disodium salt of N,N,N',N'-tetrakis-{2-[N",N"-bis-(carboxymethyl)-amino]-ethyl}-meso-2,3-diaminosuccinic acid N"',N"'-bis-(2-methoxyethyl)-diamide.

16. Metal complex according to Claim 1, that is to say the digadolinium complex of the tetrasodium salt of 1,8-bis-{4-[2-(N,N-bis-(2-(N',N'-bis-(carboxymethyl)-amino)-ethyl)-amino)-2-carboxyethyl]-phenoxy}-octane.

17. Metal complex according to Claim 1, that is to say the didysprosium complex of the tetrasodium salt of N,N'-bis-{2-[N",N"-bis-(carboxymethyl)-amino]-3-[(4-methoxy)-phenyl]-propyl}-N,N'-bis-{2-[N"'N''-bis-(carboxymethyl)-amino]-ethyl}-meso-2,3-diaminosuccinic acid.

18. Metal complex according to Claim 1, that is to say the digadolinium complex of the tetrasodium salt of 3,6,9,15,18,21-hexaaza-10,14-bis-carboxy-3,6,9,15,18,21-hexakis-(carboxymethyl)-12-oxa-tricosane-1,23-dioic acid.

19. Metal complex according to Claim 1, that is to say the didysprosium complex of the N,N,N',N'-tetrakis-{2-[[N"-(carboxymethyl)]-N"-((N"'-methyl)-carbamoylmethyl)-amino]-ethyl}-meso-2,6-diaminopimelic acid.

20. Metal complex according to Claim 1, that is to say the digadolinium complex of the tetrasodium salt of N,N,N',N'-tetrakis-{2-[N",N"-bis-(carboxymethyl)-amino]-2-(hydroxymethyl)-propyl}-mesa-2,3-diaminosuccinic acid.

21. Metal complex according to Claim 1, that is to say the digadolinium complex of the tetrasodium salt of 1,4-bis-{1-carboxy-2-[2-N,N-bis(carboxymethyl)amino-ethyl]-5,5-bis(carboxymethyl)-2,5-diazapentyl}-benzene.

22. Metal complex according to Claim 1, that is to say the diytterbium complex of the tetrasodium salt of 1,4-bis-{1-carboxy-2-[2-N,N-bis(carboxymethyl)amino-ethyl]-5,5-bis(carboxymethyl)-2,5-diazapentyl}-benzene.

23. Metal complex according to Claim 1, that is to say the dimanganese complex of the hexasodium salt of 1,4-bis-{1-carboxy-2-[2-N,N-bis(carboxymethyl)amino-ethyl]-5,5-bis(carboxymethyl)-2,5-diazapentyl}-benzene.

24. Metal complex according to Claim 1, that is to say the indium-111 complex of N,N'-bis-{2-[N",N"-bis-(carboxymethyl)-amino]-3-[(4-methoxy)-phenyl]-propyl}-N,N'-bis-{2-[N"',N"'-bis-(carboxymethyl)-amino]-ethyl}-meso-2,3-diaminosuccinic acid.

25. Metal complex according to Claim 1, that is to say the dihafnium complex of N,N,N',N'-tetrakis-{2-[N",N"-bis-(carboxymethyl)-amino]-ethyl}-2,7-diaminooctanedioic acid N"',N""-bis-(2-methoxyethyl)-diamide.

26. Metal complex according to Claim 1, that is to say the diytterbium complex of the disodium salt of N,N'-bis-{2-[N",N"-bis-(carboxymethyl)-amino]-3-[(4-ethoxy)-phenyl)-N,N'-bis-{2-[N"',N"'-bis-(carboxymethyl)-amino]-ethyl}-meso-2,3-diaminosuccinic acid N"",N""'-bis-(2-methoxyethyl)-diamide.

## Revendications

1. Composés selon la formule générale I, constitués des incréments (A) et (B): 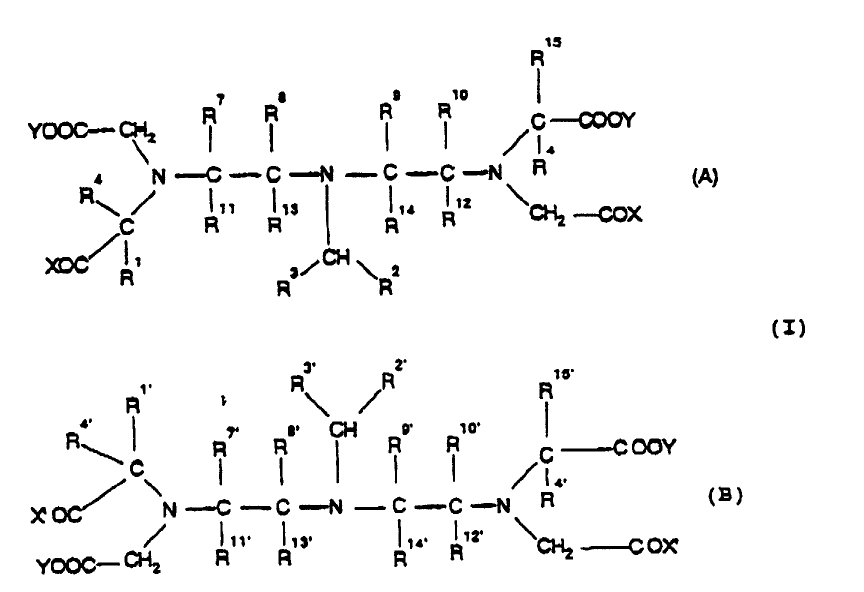
dans laquelle les incréments A et B sont reliés l'un à l'autre par l'une des paires de substituants R¹ et R^{1'}, R² et R^{2'}, R¹⁵ et R^{15'}, R¹ et R^{2'}, R^{1'} et R², R^{1'} et R¹⁵, R¹ et R^{15'}, R² et R^{15'}, R^{2'} et R¹⁵,
chacune des paires de substituants de liaison représentant un cycle hétérocyclique, un résidu phényléne, une chaîne alkylène en C₀-C₃₀ ou une chaîne aralkylène en C₇-C₃₀, qui est éventuellement substituée par 1 - 4 groupes hydroxy, alcoxy en C₁-C₆, carboxy ou mercapto et/ou est interrompue par 1 à 6 atomes d'oxygène, d'azote ou de soufre, des groupes sulfinyle et/ou sulfonyle,
et les substituants R¹, R^{1'}, R¹⁵ et R^{15'} non utilisés pour la liaison représentent indépendamment l'un de l'autre un atome d'hydrogène ou un groupe alkyle en C₁-C₆ éventuellement substitué par 1 - 4 groupes hydroxy ou mercapto,
et les substituants R² et R^{2'} non utilisés pour la liaison représentent indépendamment l'un de l'autre un atome d'hydrogène, un groupe alkyle en C₁-C₆, un groupe -(CH₂)ₙoH ou un groupe -(CH₂)ₙSH, avec n = 1 ou 2,
et les substituants R³ et R^{3'} représentent indépendamment l'un de l'autre un groupe COOY- ou un groupe CONR⁵R⁶, dans lequel R⁵ et R⁶ représentent indépendamment l'un de l'autre un groupe alkyle en C₁-C₆ qui est éventuellement substitué par 1 - 4 groupes hydroxy ou alcoxy en C₁-C₆ et/ou est interrompu par 1 - 6 atomes d'oxygène, d'azote et/ou de soufre, ou forment ensemble avec incorporation de l'atome d'azote un cycle à 5 ou 6 chaînons qui contient éventuellement un atome d'oxygène, un autre atome d'azote acylé ou un groupe sulfonyle et/ou qui est substitué avec 1 - 3 groupes hydroxy,
et les substituants R⁴ et R^{4'} représentent un atome d'hydrogène et/ou un groupe alkyle en C₁-C₆,
et les substituants R⁷-R¹⁴ et R^{7'}-R^{14'} représentent un atome d'hydrogène, un groupe phényle, une chaîne alkylène en C₀-C₃₀ ou une chaîne aralkylène en C₇-C₃₀, qui est éventuellement substituée par 1 - 4 groupes hydroxy, alcoxy en C₁-C₆ ou mercapto et/ou est interrompue par 1 - 6 atomes d'oxygène, d'azote et/ou de soufre,
ou les substituants R⁷ et R⁸, R^{7'} et R^{8'} ou R⁹ et R¹⁰ et R^{9'} et R^{10'} forment ensemble un groupe triméthylène ou tétraméthylène,
et Y représente chaque fois un atome d'hydrogène et/ou un équivalent d'ion métallique d'un élément de numéro atomique 21 - 32, 37 - 39, 42 - 51 et 57 - 83,
et X et X' représentent un groupe -OY ou -CONR⁵R⁶ dans lesquels Y, R⁵ et R⁶ ont la signification donnée plus haut,
ainsi que leurs sels avec des bases minérales et/ou organiques ou des acides aminés.

2. Composés selon la revendication 1, caractérisés en ce que Y représente un atome d'hydrogène.

3. Composés selon la revendication 1, caractérisés en ce qu'au moins deux des substituants Y représentent au moins un équivalent d'ion métallique d'un élément des nombres atomiques 21 - 32, 37 - 39, 42 - 51 et 57 - 83.

4. Composés selon la revendication 1, caractérisés en ce que la liaison entre les incréments (A) et (B) s'effectue par l'intermédiaire de l'une des paires de substituants R¹, R^{1'}; R², R^{2'}; R¹⁵, R^{15'}; R¹, R^{2'} ou R^{1'}, R².

5. Composés selon la revendication 1, caractérisés en ce que la paire de substituants R², R^{2'} représente une liaison directe, une chaîne alkylène en C₁-C_{30,} ou une chaîne aralkylène en C₇-C₃₀, qui est interrompue par deux atomes d'oxygène, ou un résidu phénylène.

6. Composés selon la revendication 1, caractérisés en ce que la paire de substituants R¹, R^{1'} représente une chaîne alkylène en C₂-C₃₀ interrompue par un atome d'oxygène.

7. Composés selon la revendication 1, caractérisés en ce que la paire de substituants R¹, R^{2'} représente une chaîne aralkylène en C₇-C₃₀ interrompue par un atome d'oxygène.

8. Agents de diagnostic contenant au moins un composé complexe selon la revendication 1, éventuellement avec les additifs habituels en galénique.

9. Procédé de préparation des composés selon la revendication 1, dans lesquels chaque Y représente au moins un équivalent d'ion métallique d'un élément des nombres atomiques 21 - 32, 37 - 39, 42 - 51 et 57 - 83, caractérisés en ce que l'on fait réagir les composés de formule I selon la revendication 1, dans lesquels Y représente un atome d'hydrogène, avec un sel métallique ou un oxyde métallique d'un élément desdits nombres atomiques.

10. Utilisation d'au moins un complexe métallique selon la revendication 1 pour la préparation d'agents de diagnostic par RMN et/ou par rayons X.

11. Utilisation d'au moins un complexe métallique selon la revendication 1 pour la préparation d'agents de radiodiagnostic et/ou de radiopthérapie.

12. Complexe métallique selon la revendication 1, à savoir le complexe digadolinium du sel tétrasodique de l'acide N,N,N',N'-tétrakis-{2-[N",N"-bis-(carboxyméthyl)amino]éthyl}-méso-2,3-diaminosuccinique.

13. Complexe métallique selon la revendication 1, à savoir le complexe digadolinium du sel tétrasodique de l'acide N,N,N',N'-tétrakis-{2-[N",N"-bis-(carboxyméthyl)amino]éthyl}méso-2,6-diaminopimélique.

14. Complexe métallique selon la revendication 1, à savoir le complexe diytterbium du sel tétrasodique de l'acide 3-{4-[2-[N,N-bis[2[N',N'-bis-(carboxyméthyl)-amino]éthyl]]amino-2-carboxyéthyl]-phénoxy}-2-{N-{2-[N'-[2-[N",N"-bis-(carboxyméthyl)-amino]éthyl]-N'-(carboxyméthyl)amino]éthyl}-N-(carboxyméthyl)amino}-propionique.

15. Complexe métallique selon la revendication 1, à savoir le complexe digadolinium du sel disodique de l'acide N,N,N',N'-tétrakis-{2-[N",N"-bis-(carboxyméthyl)amino]éthyl}méso-2,3-diaminosuccinique-N"',N""-bis-(2-méthoxyéthyl)diamide.

16. Complexe métallique selon la revendication 1, à savoir le complexe digadolinium du sel tétrasodique du 1,8-bis-{4-[2-(N,N-bis-(2-(N',N'-bis-(carboxyméthyl)amino)éthyl)amino)- 2-carboxyéthyl]-phénoxy}-octane.

17. Complexe métallique selon la revendication 1, à savoir le complexe didysprosium du sel tétrasodique de l'acide N,N'-bis-{2-[N",N"-bis-(carboxyméthyl)-amino]-3-[(4-méthoxy)phényl]propyl}-N,N'-bis-{2-[N"",N"'-bis-(carboxyméthyl)amino]éthyl}-méso-2,3-diaminosuccinique.

18. Complexe métallique selon la revendication 1, à savoir le complexe digadolinium du sel tétrasodique de 3,6,9,15,18,21-hexaaza-10,14-bis-carboxy-3,6,9,15,18,21-hexakis-(carboxyméthyl)-12-oxa-tricosane-1,23-diacide.

19. Complexe métallique selon la revendication 1, à savoir le complexe didysprosium de l'acide N,N,N',N'-tétrakis-{2-[[N"-(carboxyméthyl)]-N"-((N"'-méthyl)-carbamoylméthyl)amino]éthyl}-méso-2,6-diaminopimélique.

20. Complexe métallique selon la revendication 1, à savoir le complexe digadolinium du sel tétrasodique de l'acide N,N,N",N"-tétrakis-{2-[N'',N''-bis-(carboxyméthyl)amino]-2-(hydroxyméthyl)propyl}-méso-2,3-diaminosuccinique.

21. Complexe métallique selon la revendication 1, à savoir le complexe digadolinium du sel tetrasodique du 1,4-bis-{1-carboxy-2-[2-N,N-bis(carboxyméthyl)-aminoéthyl]-5,5-bis-(carboxyméthyl)-2,5-diazapentyl}-benzène.

22. Complexe métallique selon la revendication 1, à savoir le complexe diytterbium du sel tetrasodique du 1,4-bis-{1-carboxy-2-[2-N,N-bis-(carboxyméthyl)-aminoéthyl]-5,5-bis-(carboxyméthyl)-2,5-diazapentyl}-benzène.

23. Complexe métallique selon la revendication 1, à savoir le complexe dimanganèse du sel tetrasodique du 1,4-bis-{1-carboxy-2-[2-N,N-bis-(carboxyméthyl)-aminoéthyl]-5,5-bis-(carboxyméthyl)-2,5-diazapentyl}-benzène.

24. Complexe métallique selon la revendication 1, à savoir le complexe indium-111 de l'acide N,N'-bis-{2-[N",N"-bis-(carboxyméthyl)amino]-3-[(4-méthoxy)-phényl]propyl}-N,N'-bis-{2-[N''',N'''-bis-(carboxyméthyl)amino]éthyl}-méso-2,3-diaminosuccinique.

25. Complexe métallique selon la revendication 1, à savoir le complexe dihafnium du N"',N""-bis-(2-méthoxyéthyl)diamide du N,N,N',N'-tétrakis-{2-[N",N"-bis-(carboxyméthyl)amino]éthyl}-2,7-diaminooctanediacide.

26. Complexe métallique selon la revendication 1, à savoir le complexe diytterbium du sel disodique de l'acide N,N'-bis-{2-[N",N"-bis-(carboxyméthyl)amino]-3-[(4-éthoxy)phényl}-N,N'-bis-{2-[N"',N"'-bis-(carboxyméthyl)amino]éthyl}méso-2,3-diaminosuccinique-N"",N""'-bis-(-2-méthoxyéthyl)diamide.
